# EUROPEAN PATENT SPECIFICATION

(11) **EP 3 684 274 B1**
(45) Date of publication and mention of the grant of the patent: **09.08.2023**
(21) Application number: 18773744.0
(22) Date of filing: 19.09.2018
(51) Int. Cl.: A61B 17/3203, A61B 18/02, B24C 1/00, C01B 32/55

(54) **APPARATUS FOR MAKING DRY ICE PARTICLES, METHOD FOR OPERATING SAME, MEDICAL DRY ICE PARTICLES, METHOD FOR MAKING SAME AND USES THEREOF**
VORRICHTUNG ZUR HERSTELLUNG VON TROCKENEISPARTIKELN, VERFAHREN ZUM BETRIEB DAVON, MEDIZINISCHE TROCKENEISPARTIKEL, VERFAHREN ZUR HERSTELLUNG DAVON UND VERWENDUNGEN DAVON
APPAREIL DE FABRICATION DE PARTICULES DE GLACE SÈCHE, SON PROCÉDÉ DE FONCTIONNEMENT, PARTICULES DE GLACE SÈCHE MÉDICALES, LEUR PROCÉDÉ DE FABRICATION ET LEURS UTILISATIONS

(30) Priority: 20.09.2017 EP 17192194
(43) Date of publication of application: 29.07.2020
(73) Proprietor: aesthetiCare GmbH, 74076 Heilbronn (DE)
(72) Inventor: ROTSTEIN, Raphael, 80797 München (DE); SEIDEL, Bastian, 80336 München (DE)
(74) Representative: Ricker, Mathias
(86) International application number: PCT/EP2018/075330
(87) International publication number: WO 2019/057760

(56) References cited:
- EP-A1- 2 886 250
- WO-A1-2017/194175
- WO-A1-2019/096432
- WO-A2-02/053014
- DE-A1-102012 017 906
- DE-U1-202014 101 465
- US-A- 5 475 981
- US-A- 5 520 572

## Description

### FIELD OF THE INVENTION

The present invention relates to an apparatus for making dry ice particles, in particular for making dry ice particles for surface treatment of human and/or animal skin, to a method for operating the same and to uses thereof. Furthermore, the present invention relates to medical dry ice particles (not claimed), to a method for making same, to a method for generating a particle jet comprising dry ice particles and to uses thereof.

### BACKGROUND OF THE INVENTION

Apparatuses for making dry ice particles for surface treatment of human and/or animal tissue by using dry ice particles and methods for operating them are generally known from prior art. Particularly, surface treatment of human skin by using dry ice particles, e.g. for cleaning of the skin or for cosmetic treatment or for dermabrasion is known.

EP 1 980 365 A1 discloses an apparatus and a method for surface treatment with dry ice granulate, in particular for dermabrasion, for cosmetic treatment or for cleaning of human skin, wherein the apparatus comprises a funnel-shaped reservoir with an open top for dry ice pellets, a shredding device for shredding the pellets, an inlet for a carrier fluid flow and a mixing device with a dosing device for adding a defined amount of dry ice particles to a carrier fluid flow for generating a defined dry ice particle jet. However, because of the open-top reservoir for the dry ice pellets, a risk of contamination with pathogens exists.

DE 20 2014 101 465 U1 discloses a dry ice blasting machine for surface treatment of inorganic and organic surfaces as for example human skin, wherein this blasting machine instead of an open-top funnel-shaped reservoir for dry ice pellets comprises a storage tank for liquid carbon dioxide and is further configured for making compressed dry ice from said liquid carbon dioxide (CO2). The blasting machine further comprises a pressing die having parallel, vertical orientated channels the dry ice has to penetrate through. For shredding the compressed dry ice into small dry ice particles, the machine further comprises a milling device comprising two rollers. Furthermore, the machine comprises an inlet for supply with a pressurized carrier fluid and a mixing device with a dosing device for adding a defined amount of dry ice particles to a carrier fluid flow per time.

### OBJECTS OF THE INVENTION

In view of the above, the object of the present invention is to provide an alternative, preferably improved, apparatus for making dry ice particles and for surface treatment of human and/or animal tissue by using dry ice particles.

### SUMMARY OF THE INVENTION

This object has been achieved with an apparatus configured for making medical, in particular medical clean, dry ice particles.

The invention is defined in appended independent claims 1,8,10,12 and 13, further embodiments are described in the dependent claims. In a **first aspect** of the present invention, the invention relates to an apparatus for making dry ice particles, in particular for making dry ice particles for surface treatment of human and/or animal skin, comprising at least a first inlet port for connection to a first source of medical pressurized liquid carbon dioxide, at least a first agglomeration chamber, which is configured for making dry ice snow by delivering pressurized liquid carbon dioxide into said agglomeration chamber and expanding said pressurized liquid carbon dioxide within said agglomeration chamber, wherein the first agglomeration chamber is fluid-connectable or fluid-connected to the first inlet port, at least a first compressing device being configured for compressing the dry ice snow made in the agglomeration chamber at least partly into compressed dry ice, at least a first shredding device being configured for shredding the compressed dry ice at least partly into dry ice particles, and an outlet for discharging the dry ice particles, wherein the apparatus is configured for making medical, in particular medical clean, dry ice particles.

According to the invention, all surfaces of the apparatus being in contact and/or being configured for being in contact with substances and/or compositions for application to human and/or animal tissue by the apparatus are made of a sterilizable material anand/or are coated with sterilizable material.

In one embodiment of the invention, the at least first shredding device comprises a cutting device for cutting the compressed dry ice at least partly into dry ice particles, wherein the cutting device in particular comprises at least one cutting blade.

In one embodiment of the invention, the cutting device comprises at least one cutting roll with at least one cutting blade, wherein in particular the cutting roll is rotatable around its longitudinal axis.

According to the invention, the apparatus is further configured for generating a medical particle jet comprising a medical carrier fluid and medical dry ice particles, wherein the apparatus in particular comprises a mixing device having a mixing chamber, wherein the mixing chamber in particular comprises a fluid inlet for the medical carrier fluid flow, a particle inlet for supply with medical dry ice particles, and an outlet for discharging the particle jet generated.

In one embodiment of the invention, the apparatus further comprises at least a first and in particular a second source for medical pressurized liquid carbon dioxide and/or at least a second agglomeration chamber and/or at least a second compressing device and/or at least a second shredding device.

In one embodiment of the invention, the apparatus further comprises an application device, in particular an application device with at least a first application nozzle, wherein the application device is fluid-connectable or fluid-connected to the particle jet outlet of the mixing device and comprises a first outlet for discharging a particle jet and/or a carrier fluid flow and at least a second outlet for discharging at least a carrier fluid flow, wherein in particular the application device further comprises a switching device for switching between the first and second outlet of the application device the carrier fluid flow is discharged by.

In one embodiment of the invention, the apparatus is configured for local cryotherapy treatment of human and/or animal tissue by direct application of medical carbon dioxide and/or medical nitrogen to the tissue, wherein the apparatus in particular comprises at least one supply line, in particular a bypass line for direct fluid-connection from a source of medical liquid carbon dioxide and/or medical liquid nitrogen to the application device, in particular to at least one application nozzle.

In a **second aspect** of the present invention, the invention relates to a method for operating an apparatus defined in a **first aspect** of the present invention, comprising the steps:
- providing medical pressurized liquid carbon dioxide,
- delivering said medical pressurized liquid carbon dioxide into at least one agglomeration chamber of the apparatus and expanding said pressurized liquid carbon dioxide within the agglomeration chamber for making medical dry ice snow,
- compressing said medical dry ice snow made at least partly to medical dry ice, in particular to a medical dry ice block, and
- shredding, said compressed medical dry ice at least partly into medical dry ice particles.

In a further step a particle jet comprising a carrier fluid and medical dry ice particles is generated, in particular by adding the medical dry ice particles made at least partly into a carrier fluid flow, wherein the particle jet in particular is discharged by the outlet of the apparatus.

In a **third aspect** of the present invention, the invention relates to a method for making medical dry ice particles for surface treatment of human and/or animal tissue, in particular for surface treatment of human and/or animal skin, comprising the steps:
- providing medical pressurized liquid carbon dioxide,
- delivering said medical pressurized carbon dioxide into an agglomeration chamber and expanding the pressurized carbon dioxide for producing medical dry ice snow,
- compressing said medical dry ice snow made at least partly to medical dry ice, in particular to a medical dry ice block, and
- shredding said compressed medical dry ice at least partly into medical dry ice particles,
wherein an apparatus defined in the **first aspect** of the invention is used.

In a **fourth aspect** of the present invention, the invention relates to a method for generating a medical particle jet for surface treatment of human and/or animal tissue, in particular for surface treatment of human and/or animal skin, wherein the particle jet comprises a medical carrier fluid flow and medical dry ice particles, comprising the steps:
- making medical dry ice particles by performing a method defined in the **first aspect** of the invention and
- adding said manufactured medical dry ice particles at least partly to a medical carrier fluid flow to generate the particle jet, and
- in particular, discharging the particle jet generated by the outlet of the apparatus.

In a **fifth aspect** of the present invention, the invention relates to the use of an apparatus defined in the **first aspect** for surface treatment of human and/or animal tissue, in particular for treatment of human and/or animal skin.

In a **sixth aspect** of the present invention, the invention relates to the use of a method defined **in any one of the second to the fourth aspects** of the invention for surface treatment of human and/or animal tissue, in particular for treatment of human and/or animal skin, wherein the medical dry ice particles manufactured and/or the particle jet generated by using said method are used for the surface treatment.

In a **seventh aspect** of the present invention, which is not claimed, the invention relates to medical dry ice particles comprising medical carbon dioxide, in particular medical dry ice particles manufactured according to the method defined in the **third aspect,** for use in a method for the treatment of human and/or animal tissue, in particular for use in a method for surface treatment of human and/or animal skin.

### DETAILED DESCRIPTION OF THE INVENTION

In a **first aspect** of the present invention, the invention relates to an apparatus for making dry ice particles, in particular for making dry ice particles for surface treatment of human and/or animal skin, comprising at least a first inlet port for connection to a first source of medical pressurized liquid carbon dioxide and at least a first agglomeration chamber, which is configured for making, preferably medical, dry ice snow by delivering, preferably medical, pressurized liquid carbon dioxide into said agglomeration chamber and expanding said pressurized liquid carbon dioxide within said agglomeration chamber, wherein the first agglomeration chamber is fluid-connectable or fluid-connected to the first inlet port.

The apparatus further comprises at least a first compressing device being configured for compressing the, preferably medical, dry ice snow made in the agglomeration chamber at least partly into, preferably medical, compressed dry ice.

The apparatus further comprises at least a first shredding device being configured for shredding the, preferably medical, compressed dry ice at least partly into medical dry ice particles.

Further the apparatus comprises an outlet for discharging that medical dry ice particles.

According to the present invention, the apparatus is configured for making medical, in particular medical clean, sterile, dry ice particles of medical pressurized liquid carbon dioxide.

An apparatus according to the present invention is at least partly, preferably completely, configured for avoiding contamination of the carbon dioxide with pathogens during manufacturing of the medical dry ice particles, wherein the apparatus is preferably configured such that contamination of the carbon dioxide and/or the dry ice particles and/or other agents within the apparatus is avoided, preferably excluded.

As a consequence, an apparatus according to the present invention offers the benefit of avoiding contamination of the carbon dioxide used for making the dry ice particles. An apparatus according to the present invention further enables the possibility to use medical clean dry ice particles for surface treatment of human and/or animal tissue. By using medical dry ice particles instead of non-medical dry ice particles, the risk of infections after surface treatment of human and/or animal tissue, in particular after surface treatment of human and/or animal skin, can be reduced significantly. With an apparatus according to the present invention, in particular by using an apparatus according to the present invention, the surface treatment of human and/or animal skin can be improved.

The provision of an apparatus according to the present invention can easily, in particular with a simple construction, be realized.

In one embodiment of the present invention, all surfaces of the apparatus being in contact and/or being configured for being in contact with substances and/or compositions for application to human and/or animal tissue by the apparatus are made of a biocompatible and/or sterilizable material and/or an anti-inflammatory material and/or are coated with a biocompatible and/or sterilizable material and/or an anti-inflammatory material and/or are medical clean and/or cleanable and/or sterile and/or sterilizable.

In one embodiment of the present invention, the first inlet port of the apparatus is configured for being fluid-connected to a source of medical pressurized liquid carbon dioxide in such that contamination of the carbon dioxide with pathogens during supply of the apparatus can be avoided.

Furthermore, the at least first agglomeration chamber, the at least first compressing device and the at least first shredding device are connected, in particular to each other, in such that contamination of the carbon dioxide with pathogens during making of the dry ice particles can be avoided, in particular excluded.

The term "medical", as used herein, means suitable, in particular admitted, for medical use.

The term "medical clean", as used herein, means "clean" according to medical standards, in particular sterile, preferably substantially, in particular completely free of pathogens, wherein pathogens are, e.g. impurities, contaminates, disease agents as bacteria or viruses or toxic substances and compositions. However, "medical clean" does not mean, being free of medical additives as e.g. narcotics, antibiotics, analgesics, anti-septics or the like.

The term "dry ice particle", as used herein, refers to a solid particle at least comprising carbon dioxide (CO2) or made of carbon dioxide (CO2). The term "medical dry ice particles", as used herein, refers to dry ice particles being suitable, preferably admitted, for medical use, in particular to medical dry ice particles made by using medical admitted CO2 (carbon dioxide) comprising ≥ 99,5 Vol.-% CO2, further in particular comprising ≤ 5 ppm Volume/Volume CO (carbon monoxide), ≤ 10 ppm Volume/Volume H2O (water), in particular ≤ 1 ppm Volume/Volume sulfur in total and/or ≤ 2 ppm Volume/Volume NO/NO2 (nitrogen monoxide to nitrogen dioxide).

The term "medical pressurized liquid carbon dioxide", as used herein, refers to carbon dioxide liquefied by pressure and having a degree of purity for being classified as medical carbon dioxide and being admitted for medical use.

The term "dry ice snow", as used herein, refers to a plurality of uncompressed dry ice particles, which have not been compressed yet, i.e. to a plurality of solid particles of carbon dioxide having their original density as it appears after arising of said dry ice snow.

The term "compressed dry ice", as used herein, refers to a plurality of dry ice particles, which have been compressed, in particular by a compressing device, preferably to a dry ice block

The term "shredding device", as used herein, refers to a device being configured for shredding at least dry ice.

In one embodiment of the present invention, the medical liquid carbon dioxide can be delivered into said at least first agglomeration chamber from the top of the agglomeration chamber and/or from its lateral side, related to a functional state of use of the apparatus. In particular, the carbon dioxide can be inserted into the agglomeration chamber from above, that means from its upper side respectively its top. In another embodiment of the apparatus the carbon dioxide can be inserted laterally into the agglomeration chamber. The produced dry ice snow can preferably be collected on the bottom of the agglomeration chamber.

In one embodiment of the present invention, the at least first agglomeration chamber comprises a closable orifice for discharging the dry ice snow from the agglomeration chamber, wherein the closable orifice in particular is arranged at the bottom of the agglomeration chamber. For closing the orifice of the agglomeration chamber the apparatus preferably comprises closing means, preferably a slider, a cap, a cover plate or the like. However, by using a slider for opening and closing of the agglomeration chamber an apparatus having an easy construction can be provided. The closing means may be hydraulic actuated. Preferably, during inserting carbon dioxide into the agglomeration chamber and expanding the carbon dioxide within the agglomeration chamber the orifice for discharging the dry ice snow is closed.

In one embodiment of the present invention, the agglomeration chamber is further configured for compression of the dry ice snow by the compressing device. In particular, the dry ice snow can be compressed to dry ice within said agglomeration chamber, preferably to a dry ice block.

In one embodiment of the present invention, the compressing device comprises at least one, contrary to a reset force caused by a spring axially movable, preferably hydraulic actuated, piston for compression of the dry ice snow. The piston can be arranged for vertical or horizontal moving, related to a functional state of use of the apparatus.

In one embodiment of the present invention, an apparatus according to the present invention, in particular the compressing device, is configured for compressing the dry ice snow with a defined compression ratio.

The term "compression ratio", as used herein, refers in particular to the ratio of the volume after compression divided to the volume before compression.

In one embodiment of the present invention, the compressing device is configured for adjusting a resulting average hardness of the medical dry ice particles within a defined range. In particular, the compressing device is configured for compressing the dry ice snow such that the compressed dry ice and/or the shredded dry ice particles as a result have a hardness of at least 2 Mohs up to maximum 3 Mohs, wherein to adjust the resulting dry ice particle hardness particularly the compression ratio can be varied.

For discharging the dry ice snow, respectively the compressed dry ice, from the agglomeration chamber in an easy manner, preferably at least partly by gravity, in one embodiment according to the present invention, the agglomeration chamber and/or the compressing device are arranged above of the shredding device, i.e. on the upper side of the shredding device, related to a functional state of use of the apparatus.

In one embodiment of the present invention, the apparatus is configured for shredding the compressed dry ice at least partly, preferably completely, into medical dry ice particles with a defined particle size and/or a defined particle size distribution and/or into medical dry as particles with a defined particle form and/or with a defined particle form distribution.

The shredding device is preferably configured to shred the compressed dry ice at least partly, preferably completely, into dry ice particles with a median particle size of at least 50µm, 100µm, 150µm, 200µm, 250µm, 300µm, 350µm or 400µm up to a maximum median particle size of 100µm, 150µm, 200µm, 250µm, 300µm, 350µm, 400µm, 450µm, 500µm, 550µm, 600µm, 650µm or 700µm.

In one embodiment of the present invention, the feed rate for shredding can be adjusted at least partly or completely by gravity. In another embodiment, the apparatus comprises a feed adjusting device, particular at least one feeding piston, for achieving a constant feed rate for shredding, independent of the weight of the compressed dry ice (block)

In one embodiment of the present invention, the apparatus further comprises at least a first source for medical pressurized liquid carbon dioxide, wherein the at least first source is fluid-connectable or fluid-connected to the first inlet port, wherein said at least first source is particularly a storage volume being filled and/or being fillable with medical clean pressurized liquid carbon dioxide.

In one embodiment of the present invention, the apparatus further comprises at least a first pressure vessel being filled or being fillable with medical pressurized liquid carbon dioxide as a source, wherein said at least first pressure vessel is fluid-connectable or fluid-connected to the first inlet port.

In one embodiment of the present invention, the at least one source for medical pressurized liquid carbon dioxide is an exchangeable, common pressure gas bottle, in particular having a storage volume of at least 10 liters, preferably of at least 20 liters and in particular of maximum 50 liters for a sufficient lifetime and/or an easy exchange of the pressure vessel and/or an easy handling of the apparatus.

In one embodiment of the apparatus, the at least first shredding device comprises a cutting device for cutting the compressed dry ice at least partly, preferably completely, into medical dry ice particles, wherein the cutting device in particular comprises at least one cutting blade, in particular several cutting blades. In one embodiment of the present invention, the cutting device is configured for cutting sharp-edged medical dry ice particles.

The use of a cutting device with at least one cutting blade offers the benefit, that the shredding device, in particular the cutting device, can be cleaned, in particular sterilized, very easily. Furthermore, in a very easy manner, a cutting device, which can be disassembled easily, can be provided.

The term "cutting device", as used herein, refers to a device having at least one cutting edge being geometrically defined.

The term "cutting blade", as used herein, refers to a cutting tool with at least one geometrically defined cutting edge.

In one embodiment of the present invention, the shredding device comprises a drive unit for driving the shredding device, in particular for driving the cutting device, wherein shredding speed, in particular cutting speed, is adjustable by setting the speed of the drive unit and therewith the amount of dry ice particles shredded per time.

In one embodiment of the present invention, the cutting device comprises at least one cutting roll with at least one cutting blade, wherein in particular the cutting roll is rotatable around its longitudinal axis.

In one embodiment of the present invention, the rotatable cutting roll is arranged such that its longitudinal axis is extending horizontally or vertically in the apparatus related to a functional state of use of the apparatus. Horizontal arrangement of the cutting roll offers the benefit, that in a very easy manner two compressed dry ice blocks can be shredded next to each other simultaneously and/or consecutively. Herewith, in an easy manner, continuous shredding of medical dry ice particles can be achieved, in particular, if the apparatus is being configured for making at least two compressed dry ice (blocks) made batch wise within different agglomeration chambers.

The term "cutting roll" as used herein refers to an elongated part, which is rotatable around its longitudinal axis. Preferably, its ratio of median diameter to length (d/L) is between 1:10 and 3:1, in particular its median diameter to length ratio is of at least 0.1, 0.2, 0.3, 0.4, 0.5, 0.6, 0.7, 0.8, 0.9 or 1 up to 1.5, 2, 2.5 or 3.

The cutting roll may be shaped cylindrical or different from that. In one embodiment of the present invention, the cutting roll is cylindrical or substantially cylindrical and/or barrel-shaped.

If the cutting roll is arranged with its longitudinal axis horizontally, the cutting roll comprises preferably at least one cutting blade, in particular several cutting blades, extending at least partly, preferably completely, in radial direction of the cutting roll, preferably being arranged in circumferential direction around the lateral surface of the cutting roll.

If the cutting roll is arranged with its longitudinal axis vertically, the cutting roll preferably comprises at least one cutting blade, in particular several cutting blades, extending at least partly, preferably completely, in axial direction of the cutting roll, preferably being arranged on the top of the cutting roll, related to a functional state of use of the apparatus.

In one embodiment of the present invention, in particular if the cutting roll is arranged horizontally, preferably at least one cutting edge of at least one cutting blade is shorter than the median diameter of the corresponding cutting roll, in particular at least one cutting edge of at least one cutting blade is shorter than the median radius of the cutting roll. Preferably, the length of at least one cutting edge of at least one cutting blade is of at least 10%, 20%, 30%, 40%, 40% or 50% of the median diameter of the cutting roll up to maximum 60%, 70%, 80%, 90% or 100% of the median diameter of the cutting roll.

In one embodiment of the present invention, in particular if the cutting roll is arranged vertically, preferably at least one cutting edge of at least one cutting blade is longer than the median radius of the corresponding cutting roll, in particular at least one cutting edge of at least one cutting blade is longer than the median diameter of the cutting roll. Preferably, the length of at least one cutting edge of at least one cutting blade is of at least 50%, 60%, 70%, 80%, 90% or 100% of the median diameter of the cutting roll up to maximum 120%, 140%, 160%, 180%, 200%, 250% or 300% of the median diameter of the cutting roll.

In one embodiment of the present invention, the number of cutting blades and/or cutting depth during shredding can be varied, manually or automatically, to adjust dry ice particle geometry and/or amount of dry ice particles shredded per time.

In one embodiment of the present invention, the apparatus is further configured for generating a medical particle jet, in particular for surface treatment of human and/or animal tissue by using dry ice particles, in particular for surface treatment of human and/or animal skin, wherein the particle jet comprises a medical carrier fluid and medical dry ice particles. Therefore, the apparatus in particular comprises a mixing device having a mixing chamber, wherein the mixing chamber in particular comprises a fluid inlet for the medical carrier fluid flow, a particle inlet for supply with medical dry ice particles, and an outlet for discharging the particle jet generated.

The term "particle jet", as used herein, refers to a jet comprising particle.

The term "carrier fluid", as used herein, refers to a fluid, which is used for transporting particles, wherein the carrier fluid preferably does not react with the particles to be moved. Preferably the carrier fluid is a pressurized gas, in particular compressed-air. The carrier fluid may also be another gas than compressed air.

The term "carrier fluid flow", as used herein, refers to a flow of a carrier fluid.

In one embodiment of the present invention, the mixing chamber is configured for being flown through at least partly, preferably completely, by the carrier fluid, wherein in particular the particle jet can be generated by adding medical dry ice particles to the medical carrier fluid flow flowing through the mixing chamber.

In one embodiment of the present invention, the mixing device is arranged afterwards, in particular subsequent, to the shredding device related to flow direction of the dry ice particles. The mixing device in particular is arranged below of the shredding device, i.e. under the shredding device. Preferably, the particle inlet of the mixing device, in particular of the mixing chamber, is arranged thus the dry ice particles are able to fall into the mixing chamber by gravity.

In one embodiment of the present invention, an apparatus according to the present invention is configured for generating a particle jet for cosmetic and/or aesthetic surface treatment of human and/or animal skin.

In one embodiment of the present invention, an apparatus according to the present invention is configured for generating a particle jet for micro peeling and/or peeling and/or micro dermabrasion and/or dermabrasion of human and/or animal skin.

The term "micro peeling", as used herein, refers to a cosmetic method of ablation with small ablation depth for ablation of the upper layer of the skin at least partly, in particular by using means with a geometric indefinite cutting edge.

The term "peeling", as used herein, refers to a, in particular cosmetic, method of ablation of the upper layer of the skin, in particular by using means with a geometric indefinite cutting edge.

The term "micro dermabrasion", as used herein, refers to a cosmetic method of abrasion with small abrasion depth of the upper layer of the skin at least partly, in particular by using means with a geometric indefinite cutting edge.

The term "dermabrasion", as used herein, refers to a method of abrasion at least of the upper layer of the skin, in particular by using means with a geometric indefinite cutting edge.

In one embodiment of the present invention, medical additives, as e.g. narcotics, antibiotics, analgesics, anti-septics or the like, can be or are added to the medical (liquid) carbon dioxide and/or the dry ice particles and/or the carrier fluid flow and/or the particle jet.

In one embodiment of the present invention, the apparatus is configured for adding at least one medical additive for doting the dry ice particles and/or the particle jet, wherein preferably at least one medical additive selected from said additives listed above can be added into the agglomeration chamber for doting the dry ice snow respectively the dry ice with this additive.

In one embodiment of the present invention, the apparatus further comprises a flow generating device for generating the medical carrier fluid flow, preferably for generating a carrier fluid flow by pressurizing the carrier fluid with 0,5 to 5 bar, in particular with 1,5 to 3 bar. In particular, the flow generating device is configured for generating the carrier fluid flow from ambient air. Preferably, the flow generating device is configured for generating medical, in particular medical clean, compressed-air and a carrier fluid flow thereof.

In one embodiment of the present invention, the flow generating device comprises a suction device for sucking in ambient air, a cleaning device comprising at least one filter for cleaning the ambient air sucked in and a compressor device for pressurizing the cleaned, ambient air to create a flow of compressed-air. Flow generating devices for generating a carrier fluid flow of compressed-air for medical use are known from prior art in general.

According to the present invention, the apparatus further comprises a dosing device for adjusting the amount of dry ice particles being added to the carrier fluid flow per time, wherein the dosing device in particular comprises at least one dosing roll with a lateral surface having at least one dosing deepening arranged in said lateral surface for collecting and conveying dry ice particles, wherein the dosing roll in particular is rotatable around its longitudinal axis.

In one embodiment of the present invention, at least one dosing roll is arranged such that its longitudinal axis is extending horizontally related to a functional state of use of the apparatus. The dosing roll may also be arranged vertically or inclined.

According to the present invention, at least one dosing roll is substantially cylindrical or cylindrical and/or may be barrel-shaped or the like.

The dosing device is part of the mixing device and be arranged in the mixing chamber after the particle inlet of the mixing chamber or, not according to the invention, may be a separate part and being arranged before the mixing chamber, in particular before the particle inlet of the mixing chamber, related to particle flow direction.

According to the present invention, the dosing device is arranged horizontally within the mixing chamber, wherein the dosing roll preferably separates at least partly, preferably completely, the mixing chamber into a first and a second part, in particular in an upper and a lower part, related to a functional state of use of the apparatus. Preferably, only the second part, in particular the lower part of the mixing chamber, is configured for being flown through by the carrier fluid flow.

In one embodiment of the present invention, the fluid inlet and the particle jet outlet of the mixing chamber are arranged below the dosing roll that the lower part of the mixing chamber can be flown by the carrier fluid, while the particle inlet is arranged above the dosing roll.

According to the present invention, the apparatus is configured such that the shredded dry ice particles will fall down from the shredding device by gravity to the dosing roll, in particular into the dosing deepenings facing into the first, respectively the upper part of the mixing chamber.

In one embodiment of the present invention, the mixing device is configured that by rotation of the dosing roll, the dry ice particles collected in the dosing deepenings can be conveyed into the second, lower part of the chamber and can fall down into to the carrier fluid flow. In particular, rotation speed of the dosing roll is adjustable for adjusting the amount of dry ice particles being added to the carrier fluid flow per time to adjust particle flow rate of the particle jet. The carrier fluid flow rate may be adjustable, too. Furthermore, the dosing roll may be stopped to stop adding dry ice particles into the carrier fluid flow.

In one embodiment of the apparatus, the apparatus further comprises at least a first and in particular a second source for medical pressurized liquid carbon dioxide and/or at least a second agglomeration chamber and/or at least a second compressing device and/or at least a second shredding device. Thereby, in an easy manner, the apparatus can be configured for continuous manufacturing of medical dry ice particles, in particular for continuous shredding of medical dry ice particles and/or for continuous generating of the medical particle jet, in particular for continuous adding medical dry ice particles into the carrier fluid flow. This enables a continuous surface treatment, although dry ice snow and compressed dry ice are made batch wise.

The at least second source is particularly also a storage volume being filled and/or being fillable with medical clean pressurized, in particular liquid, carbon dioxide, preferably a pressure vessel filled or fillable with medical clean pressurized, in particular liquid, carbon dioxide, preferably also common pressure gas bottle with at least 10 liters, preferably with at least 20 liters and in particular with maximum 50 liters.

In particular, the second source is also fluid-connectable or fluid-connected to the first inlet port. The apparatus may also have a second inlet port, wherein preferably the second source of medical pressurized liquid carbon dioxide is fluid-connectable or fluid-connected to the second inlet port.

In one embodiment of the present invention, the apparatus further comprises a switching valve for selective supply with medical pressurized liquid carbon dioxide from the first source and/or the second source.

In one embodiment of the present invention, the apparatus comprises at least one control device and is configured for adjusting and/or varying operating properties according to operating requirements of the apparatus and/or application properties according to surface treatment requirements.

In one embodiment of the present invention, particle jet properties may be varied, in particular dry ice particle flow rate and/or carrier fluid flow rate and/or dry ice particle impact velocity and/or the amount of dry ice particles applied to the surface per time and/or the ratio of dry ice particle mass to carrier fluid mass per time, to achieve a defined surface treatment result.

In one embodiment of the present invention, according to the present invention, the apparatus is configured to provide a continuous particle jet and/or a pulsating particle jet, in particular a particle jet pulsating with a defined frequency, wherein the frequency can be constant or being varied, in particular depending on the surface treatment requirements. Pulsatile surface treatment may be less painful in some cases than treatment with a constant particle jet.

In one embodiment of the present invention, the apparatus further comprises an application device, in particular an application device with at least a first application nozzle, wherein the application device is fluid-connectable or fluid-connected to the particle jet outlet of the mixing device and comprises preferably a first outlet for discharging a particle jet and/or a carrier fluid flow and in particular at least a second outlet for discharging at least a carrier fluid flow.

In particular, the application device further comprises a switching device for switching between the first and second outlet of the application device to select the outlet, at least the carrier fluid flow is discharged. Thereby, in a very easy manner a very precise application of the particle jet can be achieved, because as long as the particle jet is not built up as required, the carrier fluid flow may be kept away from the surface to be treated. Herewith no dry ice particles will be applied to the surface to be treated and no surface treatment will be done. In particular, the carrier fluid flow can be kept away from the surface to be treated as long as the particle jet is built up as required.

Alternatively, and/or additionally to an application device comprising a second outlet for discharging at least the carrier fluid flow and/or the particle jet to keep it away from the surface to be treated, in one embodiment of the present invention the apparatus may further be configured for circulation of the carrier fluid flow and/or the particle jet between the application device, in particular the application nozzle, and the mixing chamber. Thereby, in an easy manner by another way, the carrier fluid flow and/or the particle jet can be kept away from the surface to be treated. Furthermore, making of the right temperature, in particular cooling, of the components being flown through by the carrier fluid flow and/or the particle jet can be achieved. This is very helpful for a precise application, in particular for building up a constant particle jet and/or for avoiding undesired sublimation of the dry ice particles in the carrier fluid flow during transport from the mixing device to the application device, in particular to the application nozzle.

Preferably, for circulation of the carrier fluid flow and/or the particle jet between the application device and the mixing chamber, the application device is fluid-connected to the mixing chamber by at least one application hose, wherein in one embodiment of an apparatus according to the present invention this application hose in particular comprises a first, inner channel for a flow from the mixing device to the application device and an outer channel, being in particular arranged circumferential, preferably coaxial, to the inner channel, for a backflow from the application device back to the mixing chamber.

In one embodiment of the present invention, the particle jet outlet of the mixing device is fluid-connectable or fluid-connected to the application device, in particular by a hose, and configured for application of the generated medical particle jet to human and/or animal tissue to be treated, preferably with the desired properties.

In one embodiment of the present invention, according to the present invention, the application device, in particular the application nozzle, preferably at least a nozzle tip, is exchangeable and/or a disposable. Thereby, the apparatus can be adapted to several application requirements, in particular due to different nozzle outlet geometries required for different applications, in a quick and easy manner. Furthermore, a disposable nozzle tips offers benefits regarding hygienic aspects.

In one embodiment of the present invention, according to the present invention, the application device, in particular the application nozzle preferably comprises a distance control device, in particular a tactile pin, which can be guided along the surface to be treated for easier moving the application nozzle equidistantly to the surface to be treated. Preferably, the effective length of that tactile pin is adjustable. Thereby, the distance control device can be adapted to different application and/or surface treatment requirements, in particular to different application distances in an easy manner.

In one embodiment of the present invention, according to the present invention, the apparatus, in particular the application nozzle, has an indication device, preferably a visual indication device and/or an acoustic indication device. In particular the apparatus comprises an indication light and/or at least one display, preferably for displaying, e.g., if the nozzle is moved within the determined distance range to the surface to be treated or not. Alternatively, and or additionally the application device may be configured for indicating a particle jet status (being build up as required for the defined treatment or not) and/or for indicating a filling level of at least one of the storage volumes.

The apparatus may further have a timer and/or counter and/or a stop watch for detecting application time, in particular for detecting duration of particle jet application to the surface. This offers a benefit for precise surface treatment because ablation depth and/or area and/or volume depends, inter alia, on time the particle jet is directed to the surface.

An apparatus according to the present invention may further be configured for application of a defined carrier fluid flow only respectively for discharging a carrier fluid flow only, i.e. without any dry ice particles and/or without any significant amount of dry ice particles, e.g. for cooling of the surface to be treated.

Therefore, in particular the dosing device can be configured in at least one operation mode of the apparatus not to add any dry ice particles to the carrier fluid flow, wherein therefore the dosing roll can preferably be stopped. Alternatively, and/or additionally, the apparatus may comprise a bypass line for circumventing the mixing device, in particular from the outlet of the flow generating device to the application device.

In one embodiment of the present invention, the apparatus is further configured for local cryotherapy treatment of human and/or animal tissue by direct application of medical carbon dioxide or medical nitrogen to the tissue, wherein the apparatus in particular comprises at least one supply line and/or at least one bypass line for direct fluid-connection from a source of medical liquid carbon dioxide and/or medical liquid nitrogen to the application device, in particular to at least one application nozzle.

The term "local cryotherapy" as used herein refers to local use of low temperatures for surface treatment, in particular in medical therapy.

An embodiment of an apparatus being configured for "local cryotherapy" as used herein is preferably at least configured for "cryosurgery" as being defined as "the application of extreme cold to destroy abnormal or diseased tissue" and/or for "ice pack therapy" as being defined as "placing ice over an injured area and is intended to absorb heat, in particular of a closed traumatic or edematous injury, by using conduction to transfer thermal energy" and/or for "cold spray anesthetics" as being defined as numbing the skin by spraying cold aerosol or the like onto the skin", in particular prior to or possibly in place of local anesthetic injections, and prior to other needles, small incisions, sutures, and so on.

The term "supply line" as used herein refers to a line or a channel which is configured for supply a device being connected by said supply line to a source with material from that source.

The term "bypass line" as used herein refers to a special supply line which is configured for bypassing at least one device. In an embodiment of an apparatus according to the present invention a bypass line for example may be configured for bypassing the agglomeration chamber and/or the compressing device and/or the mixing device.

In one embodiment of the present invention, the apparatus further comprises at least one further source for supply with medical pressurized liquid nitrogen, wherein said further source is preferably fluid-connectable or fluid-connected directly by a further supply line and/or a bypass-line to the application device, wherein said at least first source is particularly a storage volume, preferably a common pressure gas bottle, being filled and/or being fillable with medical clean pressurized liquid nitrogen.

An apparatus according to the present invention may be operated in several application modes, wherein an actual operation mode can preferably be selected by a user.

In one embodiment of the present invention, the apparatus is configured for the application of a particle jet or a carrier fluid flow only or for local cryotherapy by direct application of carbon dioxide and/or nitrogen to the surface to be treated, in particular selectively.

In one embodiment of the present invention, the apparatus may be configured for being operated in several, different operation modes, preferably in at least one predefined medical and/or at least one predefined cosmetic treatment mode and/or in at least one user-defined application mode and/or in at least one cleaning mode and/or in at least one maintenance mode.

Furthermore, an apparatus according to the invention may further comprise a scanner for scanning the surface during treatment and/or a foto- and/or a video- and/or a camera-device and/or a device for measuring the depth and/or the area and/or the volume of the surface ablation and/or abrasion.

The apparatus may also have a footswitch and/or a manual switch, several sensor devices. In particular, the apparatus at least comprises one temperature sensor for sensing surface temperature, preferably for sensing skin temperature.

In a **second aspect,** the present invention relates to a method for operating an apparatus defined in the first aspect, comprising the steps:
- providing medical pressurized liquid carbon dioxide,
- delivering said medical pressurized liquid carbon dioxide into at least one agglomeration chamber of the apparatus and expanding said pressurized liquid carbon dioxide within the agglomeration chamber for making medical dry ice snow,
- compressing said medical dry ice snow made at least partly, preferably completely, to medical dry ice, in particular to a medical dry ice block, and
- shredding, said compressed medical dry ice at least partly, preferably completely, into medical dry ice particles.

In one embodiment of the method according to the second aspect of the present invention, in a further step a particle jet comprising a carrier fluid and medical dry ice particles, is generated, in particular by adding the medical dry ice particles made at least partly to said carrier fluid flow, preferably by adding a defined amount of medical dry ice particles per time into said carrier fluid flow. In particular, the particle jet generated is discharged by the outlet of the apparatus.

In a **third aspect,** the present invention relates to a method for making medical dry ice particles for surface treatment of human and/or animal tissue, in particular for surface treatment of human and/or animal skin, comprising the steps:
- providing medical pressurized liquid carbon dioxide,
- delivering said medical pressurized carbon dioxide into an agglomeration chamber and expanding the pressurized carbon dioxide for producing medical dry ice snow,
- compressing said medical dry ice snow made at least partly, preferably completely, to medical dry ice, in particular to a medical dry ice block, and
- shredding said compressed medical dry ice at least partly, preferably completely, into medical dry ice particles,
characterized in that, that an apparatus according to any one of the claims 1 to 8 is used.

In a **fourth aspect,** the present invention relates to a method for generating a medical particle jet for surface treatment of human and/or animal tissue, in particular for surface treatment of human and/or animal skin, wherein the particle jet comprises at least a medical, particularly sterile, carrier fluid flow and medical dry ice particles, comprising the steps:
- making medical dry ice particles by performing a method defined in the **third aspect** and
- adding said manufactured medical dry ice particles at least partly, preferably completely, to a medical carrier fluid flow to generate the particle jet, in particular of a defined amount of medical dry ice particles, preferably by the dosing device, and
- in particular, discharging the particle jet generated by the outlet of the apparatus.

If the apparatus comprises at least a second agglomeration chamber and in particular at least a second compressing device and/or at least a second shredding device, dry ice snow and/or dry ice particles are made batch wise, in particular alternating in the first and the second agglomeration chamber, wherein medical dry ice particles are shredded continuously and/or are added continuously into the carrier fluid flow.

In a **fifth aspect,** the present invention relates to the use of an apparatus defined in the first aspect for surface treatment of human and/or animal tissue, in particular for treatment of human and/or animal skin.

In a **sixth aspect,** the present invention relates to the use of a method defined in the **second to fifth aspect** for surface treatment of human and/or animal tissue, in particular for treatment of human and/or animal skin, wherein the medical dry ice particles manufactured and/or the particle jet generated by using said method are used for the surface treatment.

That means, in a **sixth aspect,** the present invention relates to a method for surface treatment of human and/or animal tissue, in particular for cosmetic and/or aesthetic surface treatment of human and/or animal skin, wherein the medical dry ice particles manufactured and/or the particle jet generated by using said method defined in the **second to fifth aspect** are used for the surface treatment and in particular applied to the surface to be treated.

In one embodiment of the use according to **the sixth aspect** of the present invention, the method is used for cosmetic and/or aesthetic skin surface treatment.

In one embodiment of use according to **the sixth aspect** of the present invention, the method is used for micro peeling and/or peeling and/or micro dermabrasion and/or dermabrasion.

In one embodiment of use according to **the sixth aspect** of the present invention, a particle jet, in particular a defined particle jet, is applied for a defined time to the surface, in particular with a defined distance between skin surface and application nozzle, wherein in particular after counting down the defined time, the application of the particle jet is terminated.

In one embodiment of use according to **the sixth aspect** of the present invention, during application of the particle jet to the surface to be treated, ablation depth and/or ablation area and/or ablation area are recorded.

In one embodiment of use according to **the sixth aspect** of the present invention, during application, as long as the particle jet is not built up as required, at least the carrier fluid flow, preferably the particle jet, is kept away from the surface to be treated.

In one embodiment of use according to **the sixth aspect** of the present invention, for local cryotherapy treatment, in particular before surface treatment by using the particle jet, medical carbon dioxide and/or medical nitrogen is applied to the surface to be treated directly, in particular for cooling and/or freezing said surface, preferably by using a corresponding supply line and/or a bypass line.

In a **seventh** non-claimed **aspect,** the present invention relates to medical dry ice particles comprising medical carbon dioxide, in particular medical dry ice particles manufactured defined in the **third aspect,** for use in a method for the treatment of human and/or animal tissue, in particular for use in a method for surface treatment of human and/or animal skin.

Medical dry ice particles according to the **seventh** non-claimed **aspect** of the present invention are in particular for use in a method for micro peeling and/or for use in a method for peeling and/or for use in a method for micro dermabrasion and/or for use in a method for dermabrasion.

These and other features and advantages are apparent from the claims, as well as from the description and the following drawings, in which the individual features may be embodied on their own or in the form of sub combinations in one embodiment of the present invention.

The present invention described above, will now be further explained by the following, non-limiting embodiments and examples, wherein further optional features of the inventions are disclosed in the drawings and in the description of these drawings.

### BRIEF DESCRIPTION OF THE DRAWINGS

The accompanying drawings, which are incorporated herein and form a part of the specification, illustrate schematically preferred embodiments of the invention described above, and together with the description, serve to explain the principles and at least partly, preferably completely, the features of the described invention. The invention is explained in more detail below on the basis of a few exemplary schematically figures, wherein for same parts same reference signs are used. It is illustrated in:
- Fig. 1: a schematic illustration of a first embodiment of an apparatus according to the present invention,
- Fig. 2: a schematic illustration of the agglomeration chamber of the apparatus of Fig. 1a with a second embodiment of a compressing device,
- Fig. 3: a schematic illustration of a second embodiment of shredding device,
- Fig. 4a: a schematic illustration of an embodiment for an arrangement of two agglomeration chambers and two shredding devices for a further embodiment of an apparatus according to the present invention in a first state of use,
- Fig. 4b: a schematic illustration of the arrangement of Fig. 4a in a second state of use,
- Fig. 5: a schematic illustration of a second embodiment of an apparatus according to the present invention,
- Fig. 6: a schematic illustration of third embodiment of an apparatus according to the present the invention
- Fig. 7a: a schematic illustration of a second embodiment of an application device in a first state of use and
- Fig. 7b: a schematic illustration of the second embodiment of the application device of Fig. 7a in a second state of use.

### DETAILED DESCRIPTION OF THE DRAWINGS

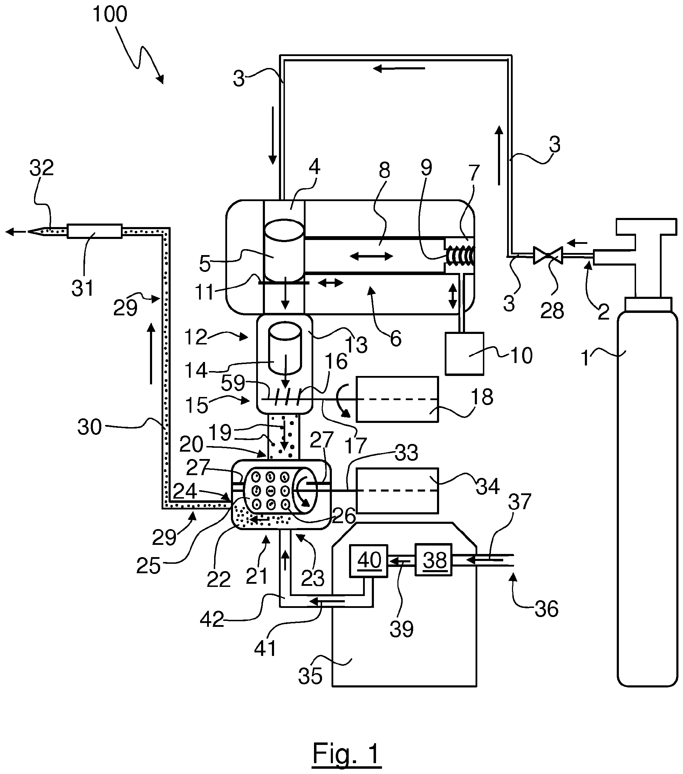

**Fig. 1** shows a first embodiment of an apparatus 100 according to the first aspect of the present invention, wherein the apparatus 100 is configured for making dry ice particles 19, in particular for making dry ice particles for surface treatment of human and/or animal skin, preferably for cosmetic and/or aesthetic skin surface treatment, e.g. for micro peeling and/or micro dermabrasion.

The apparatus 100 comprises a first inlet port 2, which is fluid-connected in a sterile manner to a first source 1 of medical (*here:* admitted for medical use) pressurized liquid carbon dioxide (CO2), stored in a common pressure gas bottle having a storage volume of 25 liters.

The apparatus 100 further comprises an agglomeration chamber 4 which is fluid-connected by supply line 3 via check valve 28 with the first inlet port 2. The agglomeration chamber 4 comprises an orifice not visualized in this illustration at its bottom, which can be opened and closed by a hydraulic actuated slider 11, which is movable in horizontal direction, related to the state of use of the apparatus 100.

If the check valve 28 is open, via supply line 3 medical pressurized liquid CO2 can be delivered into that first agglomeration chamber 4, wherein as illustrated in Fig. 1, preferably slider 11 is in the closed position during insertion of medical pressurized CO2 into the agglomeration chamber 4.

By entering the agglomeration chamber 4 the medical pressurized liquid CO2 expands and phase transformation of the CO2 occurs from liquid to solid. Thereby, dry ice snow 5 is formed. How to make dry ice snow 5 from pressurized liquid CO2 is generally known from prior art.

After some dry ice snow 5 has made, in particular after a defined amount of dry ice snow 5 has been made, the compressing device 6 can be activated for compression of the dry ice snow 5 for making compressed dry ice, wherein preferably a dry ice block 14 can be formed as illustrated symbolically in Fig. 1.

Compressing device 6 comprises a hydraulic cylinder 7, an axially movable, hydraulic piston 8 and a hydraulic drive unit 10 for actuating the piston 8 contrary to a reset force caused by spring 9. In this embodiment of an apparatus 100 the compressing device is configured and arranged that piston 8 is movable horizontally. This arrangement offers the benefit for an easy supply with the pressurized liquid CO2 into the agglomeration chamber 4 from its top.

In this embodiment, hydraulic unit 10 is configured for compressing the dry ice snow 5 with a defined compression ratio to achieve defined hardness of the compressed dry ice block 14 respectively of the dry ice particles 19 made from the compressed dry ice 14, wherein the apparatus, in particular the compressing device 6, is configured for compressing the dry ice snow 5 in that way, that the compressed dry ice 14 and/or the shredded dry ice particles 19 as a result have a hardness of at least 2 Mohs up to maximum 3 Mohs.

This embodiment of an apparatus 100 is configured for compressing the dry ice snow 5 within the agglomeration chamber 4, i.e. dry ice snow 5 can be compressed within the agglomeration chamber 4 to dry ice 14 by the compressing device 6.

In another embodiment, an apparatus according to the present invention may comprise a compressing chamber, which is separated from the agglomeration chamber 4, wherein in this case, the compressing chamber is preferably arranged below the agglomeration chamber for conveying the dry ice snow 5 made in the agglomeration chamber 4 at least partly by gravity into the compressing chamber.

By opening the orifice of the agglomeration chamber 4 at its bottom due to gravity the dry ice snow 5 respectively the compressed dry ice, in particular the compressed dry ice block 14, will be fall of the agglomeration chamber 4 downwards into shredding device 12, in particular into subsequent arranged shredding chamber 13.

This embodiment of an apparatus 100 is configured for conveying the dry ice snow 5 respectively the compressed dry ice, in particular the compressed dry ice block 14, from the agglomeration chamber 4 by gravity only. In another embodiment, an apparatus according to the present invention may comprise one or more conveying device for conveying the dry ice, e.g. a real device.

Shredding device 12, which is configured for shredding the compressed dry ice respectively the compressed dry ice block 14, at least partly, preferably completely, into dry ice particles 19, comprises a cutting device 15 with a cutting roll 59 having radial extending cutting blades 16, which are arranged at regular intervals in circumferential direction of cutting roll 59. In this embodiment, cutting roll 59 is cylinder-shaped and rotatable around its longitudinal axis 17, which is arranged horizontally. The cutting roll 59 is coupled to a cutting device drive unit 18 and can be driven by that, wherein rotational speed of the cutting roll 59 can be adjusted. The compressed dry ice to be shreeded, in particular dry ice block 14, is fed from above, in particular in vertical direction, to the cutting blades 16. With apparatus 100 the compressed medical dry ice 14 can be shredded to medical dry ice particles 19 with a size in a defined range, wherein apparatus 100 is in particular configured for shredding compressed dry ice into dry ice particles 19 with a median particle size in a range from 500µm up to 700µm.

For surface treatment of human and/or animal tissue, in particular for surface treatment of human and/or animal skin, this embodiment of an apparatus according to the present invention, i.e. apparatus 100, is further configured for generating a particle jet 29, in particular a medical particle jet 29, comprising a carrier fluid 41, in particular a medical carrier fluid 41, and medical dry ice particles 19. For discharging that particle jet 19, this apparatus 100 comprises an application device 31 with an application nozzle 32 having an outlet.

By applying a particle jet 29 comprising a carrier fluid and dry ice particles to human and/or animal tissue of cells on the tissue surface can be removed by ablation and/or abrasion. Depending on the properties of the particle jet less or more cells can be removed.

By using an apparatus according to the present invention, e.g. by using apparatus 100, and/or by performing and/or using a method according to the invention and/or by using medical dry ice particles 19 according to the invention, a medical particle jet 29 can be generated.

By using a medical particle jet 29, in particular by using an apparatus according to the present invention, e.g. by using apparatus 100, and/or by performing and/or using a method according to the invention and/or by using medical dry ice particles 19 according to the invention, for surface treatment for human and/or animal tissue, in particular for surface treatment of human and/or animal skin, the risk of infection after surface treatment of human and/or animal skin can be reduced significantly and better surface treatment results can be achieved.

For generating a particle jet 29 comprising a medical carrier fluid flow 41 and medical dry ice particles 19, the apparatus 100 comprises a mixing device 21, wherein the mixing device 21 is configured for adding medical dry ice particles 19 to a carrier fluid 41, in particular for adding medical dry ice particles 19 to a carrier fluid flow 41.

For supply of said mixing device 21 with medical dry ice particles 19, apparatus 100, in particular cutting device 15 with cutting roll 59 and cutting blades 16, is configured in such a way that the shredded dry ice particles 19 for conveying to the mixing device 21 can fall downwards by gravity into that mixing device 21. Therefore, mixing device 21 is arranged subsequent and below the shredding device 12. Thereby, in an easy manner, conveying of the shredded dry ice particles 19 from the shredding device 12 to the mixing device 21, can be realized.

In this embodiment, mixing device 21 comprises a mixing chamber 22, which comprises a first particle inlet 20 for supply with shredded medical dry ice particles 19, a carrier fluid flow inlet 23 for supply with a carrier fluid flow 41 and a particle jet outlet 24 for discharging a generated particle jet 29 from the mixing device 21.

In particular, apparatus 100 is configured such that the shredded dry ice particles 19 can fall directly into the mixing device through the particle inlet 20 of the mixing device 21. The mixing chamber 22 is configured for being partly flown through by the carrier fluid flow 41 from the inlet 23 to the particle jet outlet 24, wherein medical dry ice particles 19 can be added to the carrier fluid flow 41 in the mixing chamber 22.

For adding said medical dry ice particles 19 to the carrier fluid flow 41, in particular for adding a defined amount of particles 19 per time, the apparatus 100, in particular the mixing device 21, comprises a dosing device with a cylinder-shaped dosing roll 25 being arranged with its longitudinal axis horizontally, wherein said dosing roll 25 is rotatable around rotation axis 33 and can be driven by drive unit 34.

Medical dry ice particles 19, falling down by gravity from the shredding device 12, in particular from cutting device 15, into mixing chamber 22 through particle inlet 20 will at least partly be collected by the dosing roll 25, which comprises at its lateral surface several dosing deepenings 26. For effective collecting of the medical dry ice particles 19 by dosing roll 25 and/or for adding a defined amount of medical dry ice particles 19 precisely, in particular to avoid swirling of the medical dry ice particles 19 inserted into the mixing chamber 22 before being added to the carrier fluid flow, mixing chamber 22 is divided into two parts, in particular an upper part and a lower part, separated by dosing roll 25 and sealing means 27. Only the lower part can be flown through by the carrier fluid 41.

By rotating dosing roll 25, the medical dry ice particles 19 collected in the dosing deepenings 26 will be conveyed from the upper part of the mixing chamber 22 to the lower part of the mixing chamber 22. In particular, the medical dry ice particles will fall off the dosing deepenings 26 by gravity into the carrier fluid flow 41 flowing through the lower part of the mixing chamber 22. Thereby, the medical dry ice particles 19 are mixed with the carrier fluid flow 41 and a particle jet 29 is generated.

Apparatus 100 is further configured for generating a carrier fluid flow 41, which is necessary for generating the particle jet 29, wherein this apparatus 100 is configured for generating a carrier fluid flow 41 from ambient air. Therefore, apparatus 100 comprises a flow generating device 35 within an inlet 36 for sucking in ambient air 37. By cleaning means 38, in particular by at least one filter device 38, the ambient air 37 sucked in can be cleaned, in particular to medical clean, sterile air. By means in form of a compressor 40 the cleaned ambient air 39 can be pressurized to generate the carrier fluid flow 41. Via supply line 42 the carrier fluid, respectively the carrier fluid flow 41 can be delivered from the flow generating device 35 to the mixing device 21.

Via particle jet outlet 24 and application hose 30, which is fluid-connected to application device 31, the generated particle jet 29 can be guided from the mixing device 21 to the application device 31 with application nozzle 32. From there the particle jet can be forwarded to the outlet of the apparatus 100 for discharging of the apparatus and for surface treatment of human and/or animal tissue.

In another embodiment of an apparatus according to the present invention the apparatus may further comprise a further outlet for discharging dry ice particles. Therefore, an apparatus according to the present invention may in particular comprise an outlet for discharging dry ice particles immediately from the shredding device 12, in particular from the cutting device 15.

According to the present invention, the apparatus 100 is configured for making medical, in particular medical clean, preferably sterile dry ice particles 19. Hence, by using medical CO2, with an apparatus according to the invention medical dry ice particles 19 can be made.

All surfaces of the apparatus 100 being in contact and/or being configured for being in contact with the CO2, the dry ice snow 5, the compressed dry ice 14 and dry ice particles 19 are made of biocompatible and sterilizable material and are medical clean, in particular sterile.

Furthermore, apparatus 100 is configured for generating a medical particle jet 29 of a medical carrier fluid and medical dry ice particles 19. Therefore, also all surfaces of the apparatus 100 being in contact and/or being configured for being in contact with the carrier fluid are made of biocompatible and sterilizable material and are medical clean, in particular sterile.

In particular, apparatus 100 is configured for avoiding contamination of any of the substances or compositions appliable by the apparatus. to human and/or animal tissue. In particular, the first inlet port 2 and the pressure bottle 1 are fluid-connected such that contamination of the CO2 with pathogens within apparatus 100 can be avoided. Furthermore, the first the agglomeration chamber 4, the compressing device 6 and the shredding device 12 are also connected such that contamination of the CO2 with pathogens during making of dry ice particles 19 and/or generating particle jet 29 can be avoided, in particular excluded.

Due to the fact that results of surface treatment of human and/or animal tissue depend inter alia on the properties of the particle jet, apparatus 100 is configured for varying several particle jet properties, wherein particle jet properties among others are, e.g. particle flow rate, particle size, particle shape and carrier fluid flow rate. By varying any one of these properties, surface treatment results may be influenced, in particular tissue damage in general, ablation and/or abrasion depth and/or exactitude of particle jet application.

For precise surface treatment of human and/or animal tissue, in particular for precise surface treatment of human and/or animal skin, and/or for various applications thereof apparatus 100 is configured for varying particle flow rate, particle size and carrier fluid flow rate.

With apparatus 100, particle flow rate can be varied in particular by varying rotational speed of dosing roller 25 by varying rotational speed of dosing drive unit 34. Furthermore, particle flow rate can be influenced by varying cutting speed, by varying amount of dry ice particles 19 shredded per time and by varying size of dry ice particles 19 shredded.

For varying particle size, shredding device 12, in particular cutting device 15, is configured for varying several cutting parameters, in particular for varying cutting speed and number, geometry and arrangement of the cutting blades 16. Cutting speed can be varied by varying rotational speed of cutting drive unit 18. Number, geometry and arrangement of the cutting blades can for example being varied by using another cutting device and/or by disassembling one or more cutting blades respectively by assembling additional cutting blades with same and/or different blade geometry.

In another embodiment of an apparatus according to the present invention, cutting feed rate may also be varied and/or the cutting device may comprise, for example, one or more cutting blades configured for being activated and/or deactivated, in particular configured for being folded in and/or folded out, preferably automatically.

For varying carrier fluid flow rate, the pressure, which is applied to the cleaned carrier fluid 39 by compressor 40 for generating the carrier fluid flow 41, can be varied.

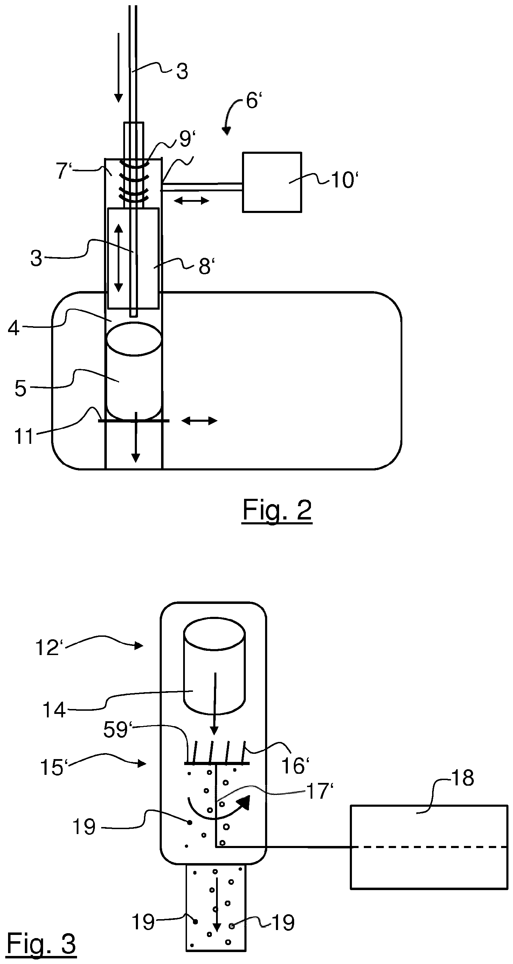

**Fig. 2** shows a schematic illustration of a second embodiment of a compressing device 6' also being configured for compressing dry ice snow 5 arranged in an agglomeration chamber 4, wherein agglomeration chamber 4 is the same as agglomeration chamber for from apparatus 100 illustrated in Fig. 1. Contrary to compression device 6 illustrated in Fig. 1, this embodiment of a compressing device 6' is configured for vertical arrangement of piston 8'.

Compressing device 6' also comprises a hydraulic cylinder 7', an axially movable, hydraulic piston 8' and a hydraulic drive unit 10' for actuating the piston 8' against a reset force caused by spring 9'. However, piston 8' is movable vertically. For an advantageously supply of the agglomeration chamber 4 with the pressurized liquid CO2, supply line 3 is at least partly arranged within piston 8' respectively runs through piston 8'.

**Fig. 3** shows a schematic illustration of a second embodiment of a shredding device 12'. This embodiment of a shredding device 12' comprises a cutting device 15', which is, contrary to cutting device 15 illustrated in Fig. 1, configured for vertical arrangement.

Cutting device 15' also comprises a cutting roll 59' having radial extending cutting blades 16', which are arranged at regular intervals in circumferential direction of cutting roll 59'. Cutting roll 59' is also cylinder-shaped and rotatable around its longitudinal axis 17'. However, cutting roll 59' is arranged vertically. Cutting roll 59' is also coupled to a cutting device drive unit 18' and can be driven by that, wherein rotational speed of the cutting roll 59' can also be adjusted. The compressed dry ice, in particular dry ice block 14, can also be fed from above.

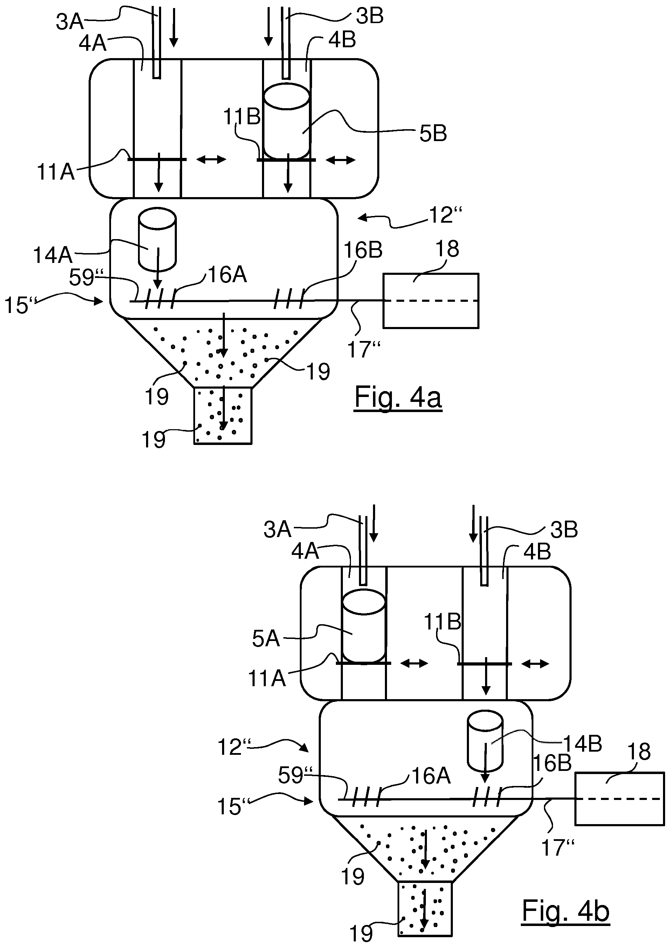

**Fig. 4a** and **4b** show a schematic illustration of an embodiment for an arrangement of two agglomeration chambers and two shredding devices for a further embodiment of an apparatus according to the present invention, wherein Fig. 4a shows the arrangement in a first state of use and Fig. 4b in a second state.

With apparatus 100 illustrated in Fig. 1, making of compressed dry ice is possible only batch wise. For this reason, the shredding device 12 may not be supplied continuously with compressed dry ice 14 for shredding. As a consequence, the cutting process may be interrupted and as a further consequence maybe not as many dry ice particles 19 as necessary can be provided for particle jet generation. As a result, the particle jet flow rate of the particle jet 29 generated may be to low or maybe no particle jet is generated. In view of the fact, that changing particle jet properties can affect surface treatment results, this is undesired. Interruption of surface treatment until new dry ice particles have been made is also undesired.

To overcome this advantage, the arrangement illustrated in Fig. 4a comprises two agglomeration chambers 4A and 4B each supplied with pressurized liquid CO2 by supply line 3A respectively 3B. The agglomeration chambers 4A and 4B are arranged next to each other, wherein the agglomeration chambers 4A and 4B each are configured as agglomeration chamber 4 of apparatus 100 illustrated in Fig. 1.

Furthermore, the arrangement comprises a shredding device 12" comprising a cutting device 15", with cutting blades 16A and 16B coupled by a common cutting roll 59" and driven by a common cutting drive unit 18'. Cutting device 15" is generally configured as cutting device 15 of apparatus 100 of Fig. 1 with the difference that cutting device 15" is configured for cutting two dry ice blocks 14.

With this arrangement illustrated in Fig. 4a and 4b, dry ice snow 5A and 5B can be made alternately and/or simultaneously in agglomeration chamber 4A and 4B and the compressed dry ice snow 14A and 14B can be shredded alternately and/or simultaneously by cutting blades 16A and 16B. This allows continuous making of dry ice particles 19. This further enables a continuous surface treatment, although dry ice snow and compressed dry ice are made batch wise. The risk of interruption of the cutting process due to lack of dry ice particles can be reduced and surface treatment can be improved.

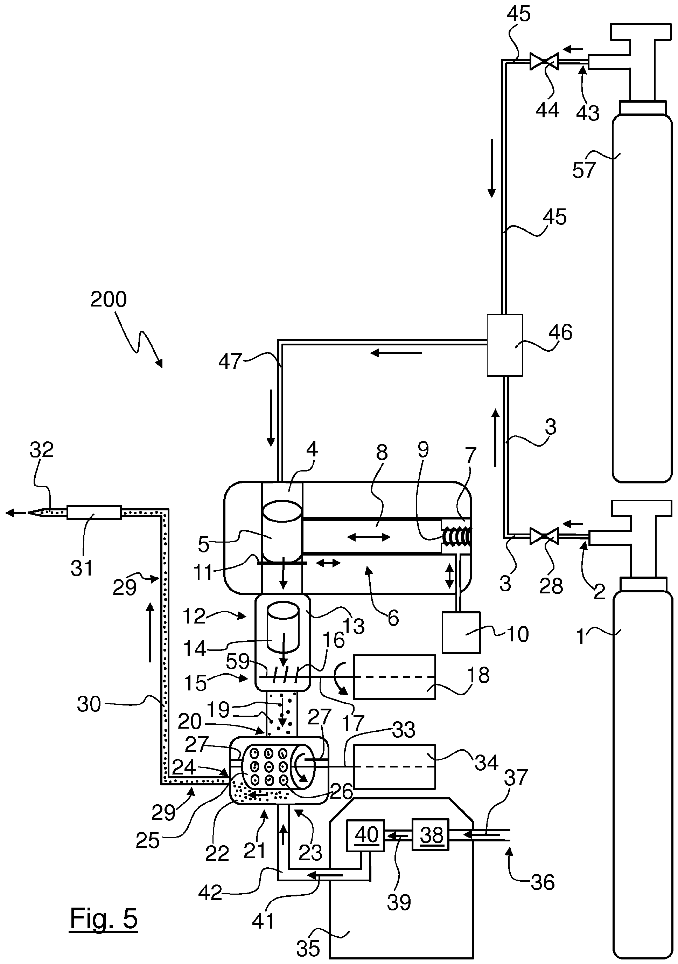

**Fig. 5** shows a schematic illustration of a second embodiment of an apparatus 200 according to the present invention, wherein this apparatus 200 compared to apparatus 100 illustrated in Fig. 1 in addition comprises a second pressure gas bottle 57 as second source for medical pressurized liquid CO2, having also a storage volume of 25 liters. For fluid-connection of the second pressure gas bottle 57 to agglomeration chamber 4, the apparatus 200 comprises a second inlet port 43.

The apparatus 200 further comprises a switching valve 46 for selective supply of agglomeration chamber 4 with medical pressurized liquid carbon dioxide from the first source 1 and/or the second source 57. Agglomeration chamber 4 can be supplied with medical pressurized liquid carbon dioxide via supply line 3, check valve 28, switching valve 46 and supply line 47 and/or via supply line 45, check valve 44, switching valve 46 and supply line 47, if the corresponding check valve 28 respectively 44 is open.

Thereby, in an easy manner, the apparatus 200 is configured for continuous manufacturing of medical dry ice particles 19 and interrupting of making medical dry ice particles 19 during exchange or refilling of the first pressure gas bottle 1 can be avoided.

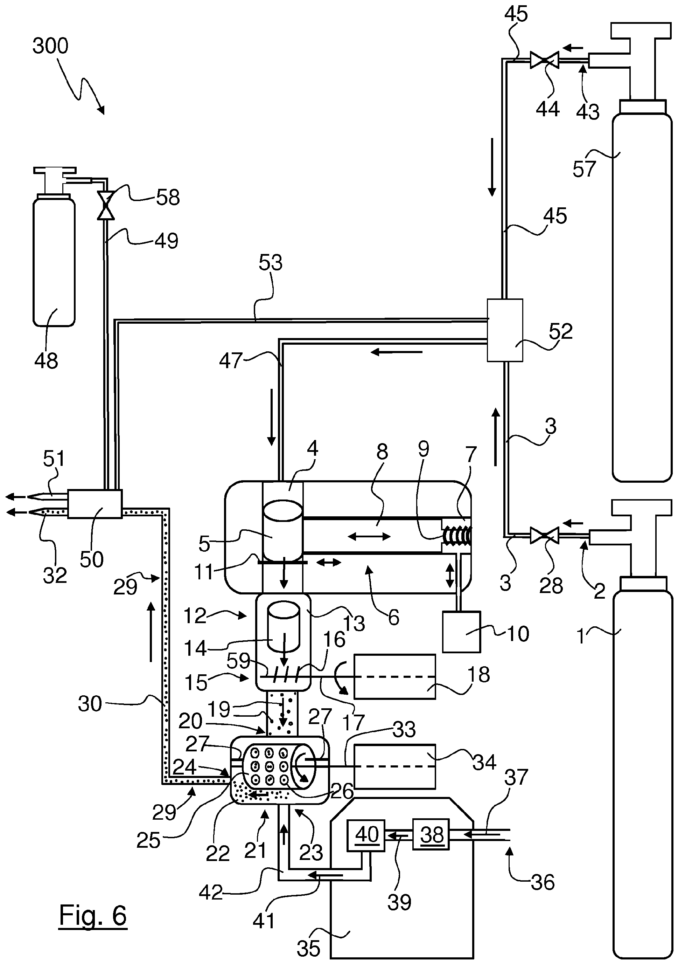

**Fig. 6** shows a schematic illustration of a third embodiment of an apparatus 300 according to the present the invention, wherein this apparatus 300 compared to apparatus 200 illustrated in Fig. 5 in addition comprises a third pressure gas bottle 48 as source for medical pressurized liquid nitrogen having a storage volume of 10 liters.

Furthermore, apparatus 300 comprises a different application device 50, which comprises a further application nozzle 51 with a further outlet, wherein application device 50, in particular application nozzle 51, is configured for local cryotherapy by using medical nitrogen and/or medical carbon dioxide.

For supply of application device 50 with said medical nitrogen for local cryotherapy, the application device 50 is immediately fluid-connected to pressure gas bottle 48 by supply line 49. For supply of application device 50 with medical carbon dioxide for local cryotherapy apparatus 300 further comprises a bypass line 53, which is for bypassing the agglomeration chamber 4, the compressing device 6, the shredding device 12, the mixing device 21 and the application hose 30.

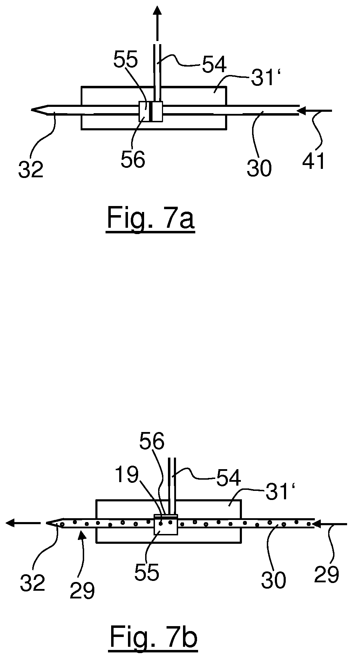

**Fig. 7a** and **7b** show a schematic illustration of a second embodiment of an application device 31', wherein Fig. 7a shows the application device 31' in a first state of use and Fig. 7b in a second state.

This application device 31' comprises an application nozzle 32 and is for example, fluid-connectable to the particle jet outlet 24 of the mixing device 21 of apparatus 100 and/or the apparatus 200. The application device 31' comprises a first outlet 32 and a second outlet 54 each for discharging a particle jet 29 and/or a carrier fluid flow 41.

The application device 31' further comprises a switching device 55 for switching between the first and second outlet 32, 54 to select the outlet, the particle jet 29 and/or the carrier fluid flow 41 is discharged by. Therefore, the switching device 55 further comprises a switching valve 56.

Due to the fact, that a practitioner is usually not able to realize that moment from that on the particle jet required for surface treatment is built up finally and has now the desired properties, better surface treatment results can be achieved, if the carrier fluid flow is kept away from the surface as long as the particle jet has not build up as defined and can be applied only after it is as defined.

This advantage can be overcome by an application device 31' as illustrated in Fig. 7a and 7b, wherein Fig. 7a shows the application device 31' in a first state, in which no particle jet has been generated already and the application device 31' is supplied with carrier fluid 41 only. In this state the first outlet 32 is closed by the switching valve 56 and the carrier fluid flow 54 is discharged by the second outlet 54.

Has the defined particle jet been built up as illustrated in Fig. 7b, the first outlet 32 can be opened by switching valve 53 and the particle jet 29 can be discharged by outlet 32 for application to the surface to be treated precisely, in particular precise for a defined time.

With an application device configured as application device 31', in a very easy manner a very precise application of the particle jet can be achieved, because with this application device, as long as the particle jet is not built up as required, the carrier fluid flow and/or the particle jet can be kept away from the surface to be treated. In particular, the carrier fluid flow can be kept away from the surface to be treated until the particle jet is built up as required. As a consequence, no dry ice particles will be applied to the surface and no surface treatment will be done for that time.

Of course, a variety of modifications are possible without departing from the scope of the claims.

### LIST OF REFERENCE SIGNS

- 100, 200, 300: apparatus according to the invention
- 1: first storage volume for medical pressurized liquid CO2
- 2: first inlet port for supply with CO2
- 3, 3A, 3B: supply line for CO2
- 4, 4A, 4B: agglomeration chamber
- 5, 5A, 5B: dry ice and snow
- 6, 6': compressing device
- 7, 7': hydraulic cylinder
- 8, 8': piston
- 9, 9': spring
- 10, 10': hydraulic driving unit
- 11, 11A, 11B: slider
- 12, 12', 12": shredding device
- 13: shredding chamber
- 14, 14A, 14B: compressed dry ice
- 15, 15', 15": cutting device
- 16, 16', 16A, 16B: cutting blades
- 17, 17', 17": rotation axis of the cutting roll
- 18: drive unit for cutting device
- 19: medical dry ice particles
- 20: particle inlet of the mixing device
- 21: mixing device
- 22: mixing chamber
- 23: carrier fluid flow inlet
- 24: particle jet outlet
- 25: dosing roll
- 26: dosing deepening
- 27: seal
- 28: check valve
- 29: particle jet
- 30: application hose
- 31, 31': application device
- 32: first application nozzle
- 33: rotation axis of the dosing roll
- 34: drive unit for dosing device
- 35: flow generating device
- 36: inlet of flow generating device
- 37: ambient air
- 38: filter device
- 39: compressor
- 40: medical clean ambient air
- 41: medical clean compressed-air
- 42: supply line for carrier fluid
- 43: second inlet port for supply with CO2
- 44: check valve
- 45: supply line for CO2
- 46: switching valve
- 47: supply line for CO2
- 48: storage volume for medical pressurized liquid nitrogen
- 49: supply line for nitrogen
- 50: application device
- 51: second application nozzle
- 52: switching valve
- 53: bypass line for CO2
- 54: second outlet of the application device
- 55: switching device
- 56: switching valve
- 57: second storage volume for medical pressurized liquid CO2
- 58: check valve
- 59, 59', 59": cutting roll

## Claims

1. Apparatus (100, 200, 300) for making sterile dry ice particles (19), in particular for making sterile dry ice particles for surface treatment of human and/or animal skin, comprising:
- at least a first inlet port (2), which is fluid-connected in a sterile manner to a first source (1) of medical pressurized liquid carbon dioxide,
- at least a first agglomeration chamber (4, 4A, 4B), which is configured for making dry ice snow (5, 5A, 5B) by delivering pressurized liquid carbon dioxide into said agglomeration chamber (4, 4A, 4B) and expanding said pressurized liquid carbon dioxide within said agglomeration chamber (4, 4A, 4B), wherein the first agglomeration chamber (4, 4A, 4B) is fluid- connectable or fluid-connected to the first inlet port (2),
- at least a first compressing device (6, 6') being configured for compressing the dry ice snow (5, 5A, 5B) made in the agglomeration chamber (4, 4A, 4B) at least partly into compressed dry ice (14, 14A, 14B),
- at least a first shredding device (12, 12', 12") being configured for shredding the compressed dry ice (14, 14A, 14B) at least partly into sterile dry ice particles (19), and
- an outlet for discharging the sterile dry ice particles (19),
wherein the apparatus is configured for making sterile dry ice particles (19) for medical use, wherein
the apparatus (100, 200, 300) comprises a mixing device (21) having a mixing chamber (22), which is divided into an upper part and a lower part, separated by a dosing roll (25) of a dosing device and sealing means (27), whereas the dosing roll (25) is cylinder-shaped and arranged with its longitudinal axis horizontally, wherein said dosing roll (25) is rotatable around rotation axis (33) and can be driven by drive unit (34), and wherein the dosing roll (25) comprises at its lateral surface several dosing deepenings (26), wherein the mixing device (21) is arranged subsequent and below the shredding device (12),
wherein the apparatus (100, 200, 300) is further configured for generating a medical particle jet (29) comprising a medical carrier fluid (41) and the medical sterile dry ice particles (19), wherein the mixing chamber (22) comprises a fluid inlet (23) for the medical carrier fluid flow (41), a particle inlet (20) for supply with medical sterile dry ice particles (19), and a particle jet outlet (24) for discharging the particle jet (29) generated, wherein the fluid inlet (23) and the particle jet outlet (24) are arranged in the lower part of the mixing chamber (22),
wherein all surfaces of the apparatus (100, 200, 300) being in contact and/or being configured for being in contact with substances and/or compositions for application to human and/or animal tissue by the apparatus (100, 200, 300) are made of a sterilizable material and/or are coated with sterilizable material.

2. Apparatus (100, 200, 300) according to claim 1, wherein all surfaces of the apparatus (100, 200, 300) being in contact and/or being configured for being in contact with substances and/or compositions for application to human and/or animal tissue by the apparatus (100, 200, 300) are made of a biocompatible material and/or are coated with a biocompatible material.

3. Apparatus (100, 200, 300) according to claim 1 or 2, wherein the at least first shredding device (12, 12', 12") comprises a cutting device (15, 15', 15") for cutting the compressed medical dry ice (14, 14A, 14B) at least partly into medical sterile dry ice particles (19), wherein the cutting device (15, 15', 15") in particular comprises at least one cutting blade (16, 16', 16A, 16B).

4. Apparatus (100, 200, 300) according to any of the preceding claims 1 to 3, wherein the cutting device (15, 15', 15") comprises at least one cutting roll (59, 59', 59") with at least one cutting blade (16, 16', 16A, 16B), wherein in particular the cutting roll (59, 59', 59") is rotatable around its longitudinal axis (17, 17', 17").

5. Apparatus (200) according to any of the preceding claims 1 to 4, wherein the apparatus (200) further comprises at least a first (1) and a second source (57) for medical pressurized liquid carbon dioxide and/or at least a second agglomeration chamber (4B) and/or at least a second compressing device (6, 6') and/or at least a second shredding device (12, 12', 12").

6. Apparatus (100, 200, 300) according to any of the preceding claims 1 to 5, wherein the apparatus (100, 200, 300) further comprises an application device (31, 31', 50), an application device (31, 31', 50) with at least a first application nozzle (32), wherein the application device (31, 31', 50) is fluid- connectable or fluid-connected to the particle jet outlet (24) of the mixing device (21) and comprises a first outlet for discharging a particle jet (29) and/or a carrier fluid flow (41) and at least a second outlet (54) for discharging at least a carrier fluid flow (41), wherein in particular the application device (31, 31', 50) further comprises a switching device (55) for switching between the first (32) and second outlet (54) of the application device (31, 31', 50) the carrier fluid flow (41) is discharged by.

7. Apparatus (300) according to any of the preceding claims 1 to 6, wherein the apparatus (300) is further configured for local cryotherapy treatment of human and/or animal tissue by direct application of medical carbon dioxide and/or medical nitrogen to the tissue, wherein the apparatus in particular comprises at least one supply line (3, 45, 47, 53, 49) as a bypass line (3, 45, 53, 49), for direct fluid-connection from a source (1, 57, 48) of medical liquid carbon dioxide and/or medical liquid nitrogen to the application device (31, 31', 50), in particular to at least one application nozzle (32, 51).

8. Method for operating an apparatus (100, 200, 300) according to claims 1 to 7, comprising the steps:
- providing medical pressurized liquid carbon dioxide,
- delivering said medical pressurized liquid carbon dioxide into at least one agglomeration chamber (4, 4A, 4B) of the apparatus (100, 200, 300) and expanding said pressurized liquid carbon dioxide within the agglomeration chamber (4, 4A, 4B) for making medical dry ice snow (5, 5A, 5B),
- compressing said medical dry ice snow (5, 5A, 5B) at least partly to medical dry ice (14, 14A, 14B), in particular to a medical dry ice block (14, 14A, 14B), and
- shredding said compressed medical dry ice (14, 14A, 14B) at least partly into medical sterile dry ice particles (19),
- collecting of the medical sterile dry ice particles by dosing roll (25),
- generating a particle jet (29) comprising a carrier fluid (41) and medical sterile dry ice particles (19), by adding the medical sterile dry ice particles (19) made at least partly to said carrier fluid flow (41).

9. Method according to claim 8, further comprising the step:
- discharging the particle jet (29) generated by the outlet of the apparatus (100, 200, 300).

10. Method for making medical sterile dry ice particles (19) for surface treatment of human and/or animal tissue, in particular for surface treatment of human and/or animal skin, comprising the steps:
- providing medical pressurized liquid carbon dioxide,
- delivering said medical pressurized carbon dioxide into an agglomeration chamber (4, 4A, 4B) and expanding the pressurized carbon dioxide for producing medical dry ice snow (5, 5A, 5B),
- compressing said medical dry ice snow (5, 5A, 5B) made at least partly to medical dry ice (14, 14A, 14B), in particular to a medical dry ice block (14, 14A, 14B), and
- shredding said compressed medical dry ice (14, 14A, 14B) at least partly into medical sterile dry ice particles (19),
- collecting of the sterile dry ice particles for medical use by dosing roll (25), wherein an apparatus (100, 200, 300) according to any one of the claims 1 to 7 is used.

11. Method for generating a medical particle jet (29) for surface treatment of human and/or animal tissue, in particular for surface treatment of human and/or animal skin, wherein the particle jet (29) comprises a medical carrier fluid flow (41) and medical sterile dry ice particles (19), comprising the steps:
- making medical sterile dry ice particles (19) by performing a method according to claim 10 and
- adding said manufactured medical sterile dry ice particles (19) at least partly into a medical carrier fluid flow (41) to generate the particle jet (29), and
- in particular, discharging by the outlet of the apparatus (100, 200, 300) the particle jet (29) generated.

12. Use of an apparatus (100, 200, 300) according to any of the claims 1 to 7 for surface treatment of human and/or animal tissue, in particular for treatment of human and/or animal skin.

13. Use of a method according to any one of claims 8 to 11 for surface treatment of human and/or animal tissue, in particular for treatment of human and/or animal skin, wherein the medical sterile dry ice particles (19) manufactured and/or the particle jet (29) generated by using said method are used for the surface treatment.

## Patentansprüche

1. Vorrichtung (100, 200, 300) zur Oberflächenbehandlung von menschlichem und/oder tierischem Gewebe unter Verwendung von Trockeneispartikeln (19), insbesondere zur Oberflächenbehandlung von menschlicher und/oder tierischer Haut, umfassend:
- mindestens eine erste Einlassöffnung (2), die mit einer ersten Quelle (1) von druckbeaufschlagtem flüssigem Kohlendioxid für medizinische Zwecke verbunden ist,
- mindestens eine erste Agglomerationskammer (4, 4A, 4B), die zum Herstellen von Trockeneisschnee (5, 5A, 5B) durch Zuführen von druckbeaufschlagtem flüssigem Kohlendioxid in die Agglomerationskammer (4, 4A, 4B) und Expandieren des druckbeaufschlagten flüssigen Kohlendioxids innerhalb der Agglomerationskammer (4, 4A, 4B) konfiguriert ist, wobei die erste Agglomerationskammer (4, 4A, 4B) fluidverbindbar oder fluidverbunden mit der ersten Einlassöffnung (2) ist,
- mindestens eine erste Verdichtungsvorrichtung (6, 6'), die konfiguriert ist, um den in der Agglomerationskammer (4, 4A, 4B) hergestellten Trockeneisschnee (5, 5A, 5B) zumindest teilweise zu komprimiertem Trockeneis (14, 14A, 14B) zu verdichten,
- mindestens eine erste Zerkleinerungsvorrichtung (12, 12', 12"), die konfiguriert ist, das komprimierte Trockeneis (14, 14A, 14B) zumindest teilweise in Trockeneispartikel (19) zu zerkleinern, und
- einen Auslass zum Ausstoßen der Trockeneispartikel (19),
wobei die Vorrichtung konfiguriert ist, um Trockeneispartikel (19) für medizinische Zwecke herzustellen, wobei
die Vorrichtung (100, 200, 300) eine Mischvorrichtung (21) mit einer Mischkammer (22) umfasst, die durch eine Dosierrolle (25) einer Dosiereinrichtung und Dichtungsmitteln (27) in einen oberen und einen unteren Teil unterteilt ist, wobei die Dosierrolle (25) zylinderförmig und mit ihrer Längsachse horizontal angeordnet ist, wobei die Dosierrolle (25) um eine Drehachse (33) drehbar und durch eine Antriebseinheit (34) antreibbar ist, und wobei
die Dosierrolle (25) an ihrer Mantelfläche mehrere Dosiervertiefungen (26) aufweist, wobei die Mischvorrichtung (21) nach und unterhalb der Zerkleinerungseinrichtung (12) angeordnet ist,
wobei die Vorrichtung (100, 200, 300) ferner zum Erzeugen eines medizinischen Partikelstrahls (29) konfiguriert ist, der ein medizinisches Trägerfluid (41) und die medizinischen sterilen Trockeneispartikel (19) umfasst, wobei die Mischkammer (22) einen Fluideinlass (23) für den medizinischen Trägerfluidstrom (41), einen Partikeleinlass (20) für die Zufuhr medizinischer steriler Trockeneispartikel (19) und einen Partikelstrahlauslass (24) zum Ableiten des erzeugten Partikelstrahls (29) umfasst,
wobei der Fluideinlass (23) und der Partikelstrahlauslass (24) im unteren Teil der Mischkammer (22) angeordnet sind,
wobei alle Oberflächen der Vorrichtung (100, 200, 300), die mit Substanzen und/oder Zusammensetzungen zur Anwendung auf menschliches und/oder tierisches Gewebe durch die Vorrichtung (100, 200, 300) in Kontakt sind und/oder dafür konfiguriert sind, mit diesen in Kontakt zu sein, aus einem sterilisierbaren Material bestehen und/oder mit sterilisierbarem Material beschichtet sind.

2. Vorrichtung (100, 200, 300) nach Anspruch 1, wobei alle Oberflächen der Vorrichtung (100, 200, 300), die mit Substanzen und/oder Zusammensetzungen zur Anwendung auf menschliches und/oder tierisches Gewebe durch die Vorrichtung (100, 200, 300) in Kontakt sind und/oder dafür konfiguriert sind, mit diesen in Kontakt zu sein, aus einem biokompatiblen Material bestehen und/oder mit einem biokompatiblen Material beschichtet sind.

3. Vorrichtung (100, 200, 300) nach Anspruch 1 oder 2, wobei die zumindest erste Zerkleinerungsvorrichtung (12, 12', 12") eine Schneidvorrichtung (15, 15', 15") zum zumindest teilweisen Zerschneiden des komprimierten medizinischen Trockeneises (14, 14A, 14B) in medizinische sterile Trockeneispartikel (19) umfasst, wobei die Schneidvorrichtung (15, 15', 15") insbesondere zumindest eine Schneidklinge (16, 16', 16A, 16B) umfasst.

4. Vorrichtung (100, 200, 300) nach einem der vorhergehenden Ansprüche 1 bis 3, wobei die Schneideinrichtung (15, 15', 15") mindestens eine Schneidwalze (59, 59', 59") mit mindestens einem Schneidmesser (16, 16', 16A, 16B) aufweist, wobei insbesondere die Schneidrolle (59, 59', 59") um ihre Längsachse (17, 17', 17") drehbar ist.

5. Vorrichtung (200) nach einem der vorhergehenden Ansprüche 1 bis 4, wobei die Vorrichtung (200) weiterhin mindestens eine erste (1) und eine zweite Quelle (57) für druckbeaufschlagtes medizinisches flüssiges Kohlendioxid und/oder mindestens eine zweite Agglomerationskammer (4B) und/oder mindestens eine zweite Verdichtungsvorrichtung (6, 6') und/oder mindestens eine zweite Zerkleinerungsvorrichtung (12, 12', 12") umfasst.

6. Vorrichtung (100, 200, 300) nach einem der vorhergehenden Ansprüche 1 bis 5, wobei die Vorrichtung (100, 200, 300) ferner eine Applikationseinrichtung (31, 31', 50) umfasst, eine Applikationseinrichtung (31, 31', 50) mit mindestens einer ersten Applikationsdüse (32), wobei die Applikationseinrichtung (31, 31', 50) mit dem Partikelstrahlauslass (24) der Mischvorrichtung (21) fluidisch verbindbar oder fluidisch verbunden ist und einen ersten Auslass zum Abgeben eines Partikelstrahls (29) und/oder eines Trägerfluidstroms (41) und mindestens einen zweiten Auslass (54) zum Abgeben mindestens eines Trägerfluidstroms (41) aufweist, wobei insbesondere die Applikationsvorrichtung (31, 31', 50) ferner eine Umschalteinrichtung (55) zum Umschalten zwischen dem ersten (32) und dem zweiten Auslass (54) der Applikationsvorrichtung (31, 31', 50) aufweist, durch die der Trägerfluidstrom (41) abgeleitet wird.

7. Vorrichtung (300) nach einem der vorhergehenden Ansprüche 1 bis 6, wobei die Vorrichtung (300) ferner zur lokalen kryotherapeutischen Behandlung von menschlichem und/oder tierischem Gewebe durch direkte Applikation von medizinischem Kohlendioxid und/oder medizinischem Stickstoff auf das Gewebe ausgebildet ist, wobei die Vorrichtung insbesondere mindestens eine Versorgungsleitung (3, 45, 47, 53, 49) als Bypass Leitung (3, 45, 53, 49) zur direkten Fluidverbindung von einer Quelle (1, 57, 48) von medizinischem Flüssigkohlendioxid und/oder medizinischem Flüssigstickstoff zu der Applikationseinrichtung (31, 31', 50), insbesondere zu mindestens einer Applikationsdüse (32, 51), aufweist.

8. Verfahren zum Betreiben einer Vorrichtung (100, 200, 300) nach einem der Ansprüche 1 bis 7, umfassend die Schritte:
- Bereitstellen von druckbeaufschlagtem flüssigem medizinischem Kohlendioxid,
- Zuführen des druckbeaufschlagten medizinischen flüssigen Kohlendioxids in mindestens eine Agglomerationskammer (4, 4A, 4B) der Vorrichtung (100, 200, 300) und Expandieren des druckbeaufschlagten flüssigen Kohlendioxids innerhalb der Agglomerationskammer (4, 4A, 4B) zur Herstellung von medizinischem Trockeneisschnee (5, 5A, 5B),
- Komprimieren des medizinischen Trockeneisschnees (5, 5A, 5B) zumindest teilweise zu medizinischem Trockeneis (14, 14A, 14B), insbesondere zu einem medizinischen Trockeneisblock (14, 14A, 14B), und
- Zerkleinern des komprimierten medizinischen Trockeneises (14, 14A, 14B) zumindest teilweise in medizinische sterile Trockeneispartikel (19),
- Sammeln der medizinischen sterilen Trockeneispartikel durch eine Dosierrolle (25),
- Erzeugen eines Partikelstrahls (29), der ein Trägerfluid (41) und medizinische sterile Trockeneispartikel (19) umfasst, durch Hinzufügen der medizinischen sterilen Trockeneispartikel (19), die zumindest teilweise zu dem Trägerfluidstrom (41) hergestellt wurden.

9. Verfahren nach Anspruch 8, das ferner den Schritt umfasst:
- Ausströmen des erzeugten Partikelstrahls (29) durch den Auslass der Vorrichtung (100, 200, 300).

10. Verfahren zur Herstellung von medizinischen sterilen Trockeneispartikeln (19) zur Oberflächenbehandlung von menschlichem und/oder tierischem Gewebe, insbesondere zur Oberflächenbehandlung von menschlicher und/oder tierischer Haut, umfassend die Schritte:
- Bereitstellen von druckbeaufschlagtem flüssigem medizinischem Kohlendioxid,
- Zuführen des druckbeaufschlagten medizinischen Kohlendioxids in eine Agglomerationskammer (4, 4A, 4B) und Expandieren des druckbeaufschlagten Kohlendioxids zur Herstellung von medizinischem Trockeneisschnee (5, 5A, 5B),
- Komprimieren des medizinischen Trockeneisschnees (5, 5A, 5B) zumindest teilweise zu medizinischem Trockeneis (14, 14A, 14B), insbesondere zu einem medizinischen Trockeneisblock (14, 14A, 14B), und
- Zerkleinern des komprimierten medizinischen Trockeneises (14, 14A, 14B) zumindest teilweise in medizinische sterile Trockeneispartikel (19),
- Sammeln der sterilen Trockeneispartikel zur medizinischen Verwendung durch eine Dosierrolle (25),
wobei eine Vorrichtung (100, 200, 300) nach einem der Ansprüche 1 bis 7 verwendet wird.

11. Verfahren zur Erzeugung eines medizinischen Partikelstrahls (29) zur Oberflächenbehandlung von menschlichem und/oder tierischem Gewebe, insbesondere zur Oberflächenbehandlung von menschlicher und/oder tierischer Haut, wobei der Partikelstrahl (29) einen medizinischen Trägerfluidstrom (41) und medizinische sterile Trockeneispartikel (19) umfasst, umfassend die Schritte:
- Herstellen von medizinischen sterilen Trockeneispartikeln (19) durch Ausführen eines Verfahrens nach Anspruch 10 und
- Hinzufügen der hergestellten medizinischen sterilen Trockeneispartikel (19) zumindest teilweise in einen medizinischen Trägerfluidstrom (41), um den Partikelstrahl (29) zu erzeugen, und
- insbesondere Ausströmen lassen des erzeugten Partikelstrahls (29) durch den Auslass der Vorrichtung (100, 200, 300).

12. Verwendung einer Vorrichtung (100, 200, 300) nach einem der Ansprüche 1 bis 7 zur Oberflächenbehandlung von menschlichem und/oder tierischem Gewebe, insbesondere zur Behandlung von menschlicher und/oder tierischer Haut.

13. Verwendung eines Verfahrens nach einem der Ansprüche 8 bis 11 zur Oberflächenbehandlung von menschlichem und/oder tierischem Gewebe, insbesondere zur Behandlung von menschlicher und/oder tierischer Haut, wobei die hergestellten medizinisch sterilen Trockeneispartikel (19) und/oder der durch Anwendung des Verfahrens erzeugte Partikelstrahl (29) zur Oberflächenbehandlung verwendet werden.

## Revendications

1. Appareil (100, 200, 300) pour la fabrication de particules de glace sèche stériles (19), en particulier pour la fabrication de particules de glace sèche stériles pour un traitement de surface de la peau humaine et/ou animale, comprenant :
- au moins un premier orifice d'entrée (2), qui est relié fluidiquement de manière stérile à une première source (1) de dioxyde de carbone liquide sous pression à usage médical,
- au moins une première chambre d'agglomération (4, 4A, 4B), qui est configurée pour fabriquer de la neige carbonique (5, 5A, 5B) par distribution de dioxyde de carbone liquide sous pression dans ladite chambre d'agglomération (4, 4A, 4B) et par détente dudit dioxyde de carbone liquide sous pression dans ladite chambre d'agglomération (4, 4A, 4B), dans lequel la première chambre d'agglomération (4, 4A, 4B) est apte à être reliée fluidiquement ou est reliée fluidiquement au premier orifice d'entrée (2),
- au moins un premier dispositif de compression (6, 6') configuré pour comprimer la neige carbonique (5, 5A, 5B) fabriquée dans la chambre d'agglomération (4, 4A, 4B) au moins partiellement en glace sèche comprimée (14, 14A, 14B),
- au moins un premier dispositif de broyage (12, 12', 12") configuré pour broyer la glace sèche comprimée (14, 14A, 14B) au moins partiellement en particules de glace sèche stériles (19), et
- une sortie pour évacuer les particules de glace sèche stériles (19),
dans lequel l'appareil est configuré pour fabriquer des particules de glace sèche stériles (19) à usage médical,
dans lequel l'appareil (100, 200, 300) comprend un dispositif de mélange (21) ayant une chambre de mélange (22), qui est divisée en une partie supérieure et une partie inférieure, séparées par un rouleau de dosage (25) d'un dispositif de dosage et des moyens d'étanchéité (27), tandis que le rouleau de dosage (25) est en forme de cylindre et est agencé avec son axe longitudinal s'étendant horizontalement, dans lequel ledit rouleau de dosage (25) est apte à pivoter autour d'un axe de rotation (33) et peut être entraîné par une unité d'entraînement (34), et dans lequel le rouleau de dosage (25) comprend plusieurs cavités de dosage (26) au niveau de sa surface latérale, dans lequel le dispositif de mélange (21) est disposé de manière subséquente et au-dessous du dispositif de broyage (12),
dans lequel l'appareil (100, 200, 300) est en outre configuré pour générer un jet de particules à usage médical (29) comprenant un fluide porteur à usage médical (41) et les particules de glace sèche stériles à usage médical (19), dans lequel la chambre de mélange (22) comprend une entrée de fluide (23) pour l'écoulement du fluide porteur à usage médical (41), une entrée de particules (20) pour l'alimentation en particules de glace sèche stériles à usage médical (19), et une sortie de jet de particules (24) pour l'évacuation du jet de particules (29) généré,
dans lequel l'entrée de fluide (23) et la sortie de jet de particules (24) sont disposées dans la partie inférieure de la chambre de mélange (22),
dans lequel toutes les surfaces de l'appareil (100, 200, 300) en contact et/ou configurées pour être en contact avec des substances et/ou des compositions destinées à une application sur un tissu humain et/ou animal par l'appareil (100, 200, 300) sont constituées d'un matériau stérilisable et/ou sont revêtues d'un matériau stérilisable.

2. Appareil (100, 200, 300) selon la revendication 1, dans lequel toutes les surfaces de l'appareil (100, 200, 300) en contact et/ou configurées pour être en contact avec des substances et/ou des compositions destinées à une application sur un tissu humain et/ou animal par l'appareil (100, 200, 300) sont constituées d'un matériau biocompatible et/ou sont revêtues d'un matériau biocompatible.

3. Appareil (100, 200, 300) selon la revendication 1 ou 2, dans lequel l'au moins un premier dispositif de broyage (12, 12', 12") comprend un dispositif de coupe (15, 15', 15") pour couper la glace sèche comprimée à usage médical (14, 14A, 14B) au moins partiellement en particules de glace sèche stériles à usage médical (19), dans lequel le dispositif de coupe (15, 15', 15") comprend en particulier au moins une lame de coupe (16, 16', 16A, 16B).

4. Appareil (100, 200, 300) selon l'une quelconque des revendications précédentes 1 à 3, dans lequel le dispositif de coupe (15, 15', 15") comprend au moins un rouleau de coupe (59, 59', 59") avec au moins une lame de coupe (16, 16', 16A, 16B), dans lequel en particulier le rouleau de coupe (59, 59', 59") est apte à pivoter autour de son axe longitudinal (17, 17', 17").

5. Appareil (200) selon l'une quelconque des revendications précédentes 1 à 4, dans lequel l'appareil (200) comprend en outre au moins une première (1) et une deuxième source (57) de dioxyde de carbone liquide sous pression à usage médical et/ou au moins une deuxième chambre d'agglomération (4B) et/ou au moins un deuxième dispositif de compression (6, 6') et/ou au moins un deuxième dispositif de broyage (12, 12', 12").

6. Appareil (100, 200, 300) selon l'une quelconque des revendications précédentes 1 à 5, dans lequel l'appareil (100, 200, 300) comprend en outre un dispositif d'application (31, 31', 50), un dispositif d'application (31, 31', 50) avec au moins une première buse d'application (32), dans lequel le dispositif d'application (31, 31', 50) est apte à être relié fluidiquement ou relié fluidiquement à la sortie de jet de particules (24) du dispositif de mélange (21) et comprend une première sortie pour évacuer un jet de particules (29) et/ou un écoulement de fluide porteur (41) et au moins une deuxième sortie (54) pour évacuer au moins un écoulement de fluide porteur (41), dans lequel, en particulier, le dispositif d'application (31, 31', 50) comprend en outre un dispositif de commutation (55) pour commuter entre la première (32) et la deuxième sortie (54) du dispositif d'application (31, 31', 50) duquel l'écoulement de fluide porteur (41) est évacué.

7. Appareil (300) selon l'une quelconque des revendications précédentes 1 à 6, dans lequel l'appareil (300) est en outre configuré pour un traitement local de cryothérapie de tissu humain et/ou animal par application directe de dioxyde de carbone à usage médical et/ou d'azote à usage médical sur le tissu, dans lequel l'appareil comprend en particulier au moins une ligne d'alimentation (3, 45, 47, 53, 49) en tant que ligne de dérivation (3, 45, 53, 49), pour connexion fluidique directe d'une source (1, 57, 48) de dioxyde de carbone liquide à usage médical et/ou d'azote liquide à usage médical au dispositif d'application (31, 31', 50), en particulier à au moins une buse d'application (32, 51).

8. Procédé de fonctionnement d'un appareil (100, 200, 300) selon les revendications 1 à 7, comprenant les étapes suivantes :
- la fourniture de dioxyde de carbone liquide sous pression à usage médical,
- la distribution dudit dioxyde de carbone liquide sous pression à usage médical dans au moins une chambre d'agglomération (4, 4A, 4B) de l'appareil (100, 200, 300) et la détente dudit dioxyde de carbone liquide sous pression dans la chambre d'agglomération (4, 4A, 4B) pour fabriquer de la neige carbonique à usage médical (5, 5A, 5B),
- la compression de ladite neige carbonique à usage médical (5, 5A, 5B) au moins partiellement en glace sèche à usage médical (14, 14A, 14B), en particulier en un bloc de glace sèche à usage médical (14, 14A, 14B), et
- le broyage de ladite glace sèche à usage médical comprimée (14, 14A, 14B) au moins partiellement en particules de glace sèche stériles à usage médical (19),
- la collecte des particules de glace sèche stériles à usage médical par un rouleau de dosage (25),
- la génération d'un jet de particules (29) comprenant un fluide porteur (41) et des particules de glace sèche stériles à usage médical (19), par ajout des particules de glace sèche stériles à usage médical (19) fabriquées au moins partiellement audit écoulement de fluide porteur (41).

9. Procédé selon la revendication 8, comprenant en outre l'étape suivante :
- l'évacuation du jet de particules (29) généré par la sortie de l'appareil (100, 200, 300).

10. Procédé de fabrication de particules de glace sèche stériles à usage médical (19) pour un traitement de surface de tissu humain et/ou animal, en particulier pour un traitement de surface de la peau humaine et/ou animale, comprenant les étapes suivantes :
- la fourniture de dioxyde de carbone liquide sous pression à usage médical,
- la distribution dudit dioxyde de carbone sous pression à usage médical dans une chambre d'agglomération (4, 4A, 4B) et la détente du dioxyde de carbone sous pression pour produire de la neige carbonique à usage médical (5, 5A, 5B),
- la compression de ladite neige carbonique à usage médical (5, 5A, 5B) fabriquée au moins partiellement en glace sèche à usage médical (14, 14A, 14B), en particulier en un bloc de glace sèche à usage médical (14, 14A, 14B), et
- le broyage de ladite glace sèche à usage médical comprimée (14, 14A, 14B) au moins partiellement en particules de glace sèche stériles à usage médical (19),
- la collecte des particules de glace sèche stériles à usage médical par un rouleau de dosage (25),
dans lequel un appareil (100, 200, 300) selon l'une quelconque des revendications 1 à 7 est utilisé.

11. Procédé de génération d'un jet de particules à usage médical (29) pour un traitement de surface de tissu humain et/ou animal, en particulier pour un traitement de surface de la peau humaine et/ou animale, dans lequel le jet de particules (29) comprend un écoulement de fluide porteur à usage médical (41) et des particules de glace sèche stériles à usage médical (19), comprenant les étapes suivantes :
- la fabrication de particules de glace sèche stérile à usage médical (19) par la mise en oeuvre d'un procédé selon la revendication 10 et
- l'ajout desdites particules de glace sèche stériles à usage médical (19) fabriquées au moins partiellement dans un écoulement de fluide porteur à usage médical (41) pour générer le jet de particules (29), et
- en particulier, l'évacuation par la sortie de l'appareil (100, 200, 300) du jet de particules (29) généré.

12. Utilisation d'un appareil (100, 200, 300) selon l'une quelconque des revendications 1 à 7 pour un traitement de surface de tissu humain et/ou animal, en particulier pour un traitement de la peau humaine et/ou animale.

13. Utilisation d'un procédé selon l'une quelconque des revendications 8 à 11 pour un traitement de surface de tissu humain et/ou animal, en particulier pour un traitement de la peau humaine et/ou animale, dans laquelle les particules de glace sèche stériles à usage médical (19) fabriquées et/ou le jet de particules (29) généré à l'aide dudit procédé sont utilisés pour le traitement de surface.
